(19) Europäisches Patentamt
European Patent Office
Office européen des brevets

(11) Veröffentlichungsnummer: **0 111 873**
**B1**

(12) # EUROPÄISCHE PATENTSCHRIFT

(45) Veröffentlichungstag der Patentschrift: **07.10.87**

(51) Int. Cl.⁴: **C 07 D 209/52**, C 07 K 5/06, A 61 K 31/40

(21) Anmeldenummer: **83112506.7**

(22) Anmeldetag: **13.12.83**

(54) **Derivate der cis, endo-2-Azabicyclo-(5.3.0)-decan-3-carbonsäure, Verfahren zu ihrer Herstellung, diese enthaltende Mittel und deren Verwendung.**

(30) Priorität: **16.12.82 DE 3246503**

(43) Veröffentlichungstag der Anmeldung: **27.06.84 Patentblatt 84/26**

(45) Bekanntmachung des Hinweises auf die Patenterteilung: **07.10.87 Patentblatt 87/41**

(84) Benannte Vertragsstaaten: **AT BE CH DE FR GB IT LI LU NL SE**

(56) Entgegenhaltungen:
**EP - A - 0 037 231**
**EP - A - 0 050 800**
**EP - A - 0 079 022**
**EP - A - 0 079 522**
**EP - A - 0 081 094**
**EP - A - 0 084 164**
**EP - A - 0 090 362**
**US - A - 3 113 950**

**Arzneimit.-Forsch/Drug Res. 34 (II) Nr. 106 (1984) 1411-1416**

**Die Akte enthält technische Angaben, die nach dem Eingang der Anmeldung eingereicht wurden und die nicht in dieser Patentschrift enthalten sind.**

(73) Patentinhaber: **HOECHST AKTIENGESELLSCHAFT, Postfach 80 03 20, D-6230 Frankfurt am Main 80 (DE)**

(72) Erfinder: **Urbach, Hansjörg, Dr., Le Lavandoustrasse 41, D-6242 Kronberg/Taunus (DE)**
Erfinder: **Henning, Rainer, Dr., Rotenhofstrasse 31, D-6234 Hattersheim am Main (DE)**
Erfinder: **Teetz, Volker, Dr., An der Tann 20, D-6238 Hofheim am Taunus (DE)**
Erfinder: **Becker, Reinhard, Dr., Adelheidstrasse 101, D-6200 Wiesbaden (DE)**

EP 0 111 873 B1

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

**Beschreibung**

«Aus EP-A 37 231 sind Octahydro-1H-indol-2-carbonsäure-Derivate bekannt. Diese besitzen blutdrucksenkende Wirkung.

Die europäische Patentanmeldung EP-A 50 800 betrifft Carboxyalkyl-Dipeptide und EP-A 84 164 cis,exo- und trans-Verbindungen bicyclischer Aminosäure-Derivate. Es werden ausserdem Verfahren zu ihrer Herstellung beschrieben sowie deren Verwendung als ACE-Inhibitoren.»

Die Erfindung betrifft Derivate der cis, endo-2-Azabicyclo-[5.3.0]-decan-3-carbonsäure der Formel I

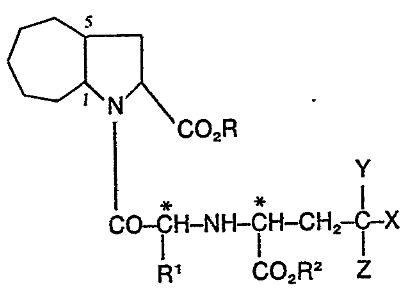

(I)

in der die Wasserstoffatome an den Brückenkopf-C-Atomen 1 und 5 zueinander cis- konfiguriert sind und die Carboxygruppe am C-Atom 3 endoständig zum bicyclischen Ringsystem orientiert ist und worin

R = Wasserstoff, $(C_1–C_6)$-Alkyl, $(C_2–C_6)$-Alkenyl oder $(C_6–C_{12})$-Aryl-$(C_1–C_4)$-alkyl,

$R^1$ = Wasserstoff, Allyl, Vinyl oder eine Seitenkette einer gegebenenfalls geschützten natürlich vorkommenden α-Aminosäure $R^1$–CH($NH_2$)–COOH,

$R^2$ = Wasserstoff, $(C_1–C_6)$-Alkyl, $(C_2–C_6)$-Alkenyl oder $(C_6–C_{12})$-Aryl-$(C_1–C_4)$-alkyl,

Y = Wasserstoff oder Hydroxy,

Z = Wasserstoff oder

Y und Z = zusammen Sauerstoff,

X = $(C_1–C_6)$-Alkyl, $(C_2–C_6)$-Alkenyl, $(C_5–C_9)$-Cycloalkyl, $(C_6–C_{12})$-Aryl, vorzugsweise Phenyl, das durch $(C_1–C_4)$-Alkyl, $(C_1–C_4)$-Alkoxy, Hydroxy, Halogen, Nitro, Amino, $(C_1–C_4)$-Alkylamino, Di-$(C_1–C_4)$-alkylamino oder Methylendioxy mono-, di- oder trisubstituiert sein kann, oder Indol-3-yl bedeuten; sowie deren physiologisch unbedenkliche Salze.

Bevorzugt sind Verbindungen der Formel I, in der

R = Wasserstoff,

$R^1$ = Methyl, die gegebenenfalls acylierte Seitenkette von Lysin oder die O-alkylierte Seitenkette des Tyrosin,

$R^2$ = Wasserstoff, Methyl, Ethyl, Benzyl oder tert. Butyl,

X = Phenyl oder mit Fluor und/oder Chlor mono- oder disubstituiertes Phenyl,

Y = Wasserstoff oder Hydroxy,

Z = Wasserstoff oder

Y und Z zusammen Sauerstoff bedeuten.

Unter Aryl wird hier im folgenden vorzugsweise Phenyl verstanden.

Als besonders bevorzugte Verbindungen seien erwähnt:

N-(1-S-Carbethoxy-3-phenyl-propyl)-S-alanyl-cis,endo-2-azabicyclo-[5.3.0]-decan-3-S-carbonsäure,

N-(1-S-Carboxy-3-phenyl-propyl)-S-alanyl-cis,endo-2-azabicyclo-[5.3.0]-decan-3-S-carbonsäure,

N-(1-S-Carbethoxy-3-phenyl-propyl)-S-lysyl-cis,endo-2-azabicyclo-[5.3.0]-decan-3-S-carbonsäure,

N-(1-S-Carboxy-3-phenyl-propyl)-S-lysyl-cis,endo-2-azabicyclo-[5.3.0]-decan-3-S-carbonsäure,

N-(1-S-Carbethoxy-3-phenyl-propyl)-O-ethyl-S-tyrosyl-cis,endo-2-azabicyclo-[5.3.0]-decan-3-S-carbonsäure

und N-(1-S-Carbethoxy-3-phenyl-propyl)-O-methyl-S-tyrosyl-cis,endo-2-azabicyclo-[5.3.0]-decan-3-S-carbonsäure.

Falls $R^1$ für eine Seitenkette einer geschützten natürlich vorkommenden α-Aminosäure steht, wie z. B. geschütztes Ser, Thr, Asp, Asn, Glu, Gln, Arg, Lys, Hyl, Cys, Orn, Cit, Tyr, Trp, His oder Hyp, sind als Schutzgruppen die in der Peptidchemie üblichen Gruppen bevorzugt (vgl. Houben-Weyl, Bd. XV/1 und XV(2)). Im Falle, dass $R^1$ die geschützte Lysin-Seitenkette bedeutet, werden die bekannten Aminoschutzgruppen, insbesondere aber $(C_1–C_6)$-Alkanoyl bevorzugt. Im Falle, dass $R^1$ die geschützte Tyrosin-Seitenkette bedeutet, wird die Ether-Schutzgruppe am Sauerstoff, insbesondere $(C_1–C_6)$-Alkyl, bevorzugt; besonders bevorzugte Schutzgruppen sind Methyl und Ethyl.

Als Salze kommen insbesondere in Frage die Hydrochloride, Maleinate, Tartrate bzw. die Alkali-, Ca-, Mg- und Zn-Salze.

Die Chiralitätszentren an den mit einem Stern (*) markierten C-Atomen der Kette und an C-Atom 3 des Bicyclus können sowohl die R- als auch die S-Konfiguration haben. Bevorzugt sind jedoch Verbindungen, in denen diese Zentren in der S-Konfiguration vorliegen, mit der Ausnahme, dass bei (NH–CH$R^1$–CO) = Cys die R-Konfiguration dieses Zentrums bevorzugt ist.

Die Erfindung betrifft ferner ein Verfahren zur Herstellung der Verbindungen der Formel I, das dadurch gekennzeichnet ist, dass man eine Verbindung der Formel II, in der $R^2$ die vorstehend genannten Bedeutungen mit Ausnahme der von Wasserstoff hat, mit einer Verbindung der Formeln IIIa oder IIIb oder dem Racemat, in denen W eine Carboxy veresternde Gruppe, wie $(C_1–C_6)$-Alkyl oder $(C_7–C_8)$-Aralkyl, vorzugsweise tert.-Butyl oder Benzyl bedeutet, nach bekannten Amidbildungsmethoden der Peptidchemie zu Verbindungen der Formel I umsetzt, diese ggfs. durch Hydrierung, Säure- oder/und Basenbehandlung in Verbindungen der Formel I (R und/oder $R^2$ = H) überführt, so erhaltene Verbindungen ggfs. in an sich bekannter Weise verestert und/oder ggfs. in ihre physiologisch unbedenklichen Salze überführt.

Diastereomere der Formel I können beispielsweise durch Kristallisation oder Chromatographie voneinander getrennt werden.

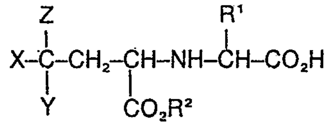
(II)

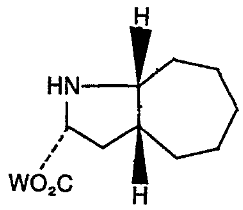
(IIIa)

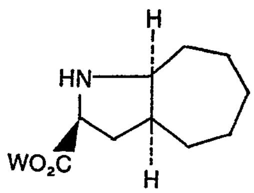
(IIIb)

Verbindungen der Formel II sind bereits vorgeschlagen worden. Solche mit X = Phenyl, Y = H, Z = H, $R^1 = CH_3$ und $R^2 = CH_3$ oder $C_2H_5$ sind bekannt (z.B. aus der Ep-Schr. 0 037 231) und auf verschiedenen Wegen zugänglich. Die Benzylester ($R^2$ = Benzyl) können analog hergestellt werden.

Die Mannich-Reaktion von Acetophenonen der Formeln IVa, in der X für gegebenenfalls wie vorstehend substituiertes Aryl steht, mit Glyoxylsäureestern und α-Aminosäureestern führt zu Verbindungen der Formel II, in denen Y und z zusammen Sauerstoff bedeuten (Formel IV). In der Formel IV bedeutet W′ einen hydrogenolytisch, basisch oder sauer abspaltbaren Rest, bevorzugt Benzyl oder tert. Butyl, X steht für gegebenenfalls wie vorstehend definierten Bedeutungen. Im Falle des Benzylesters (W′ = Benzyl) darf jedoch $R^2$ nicht Benzyl sein. Bei der Hydrogenolyse dieser Verbindungen mit Pd entstehen Verbindungen der Formel II, in denen Y und Z Wasserstoff sind.

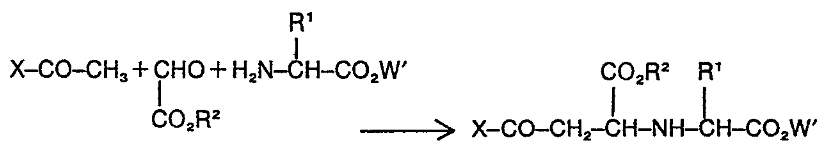
(IVa) (IV)

Verbindungen der Formel II in der Y und Z zusammen Sauerstoff bedeuten, können ebenfalls durch Michael-Addition entsprechender Keto-Acrylsäureester mit α-Aminosäureestern in hohen Ausbeuten gewonnen werden. Esterspaltung führt zu denselben Produkten wie die Mannich-Reaktion.

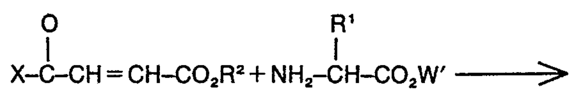
(IV)

Die Diastereomeren mit der bevorzugten S,S-Konfiguration entstehen bei Einsatz von L-Alaninestern in überwiegender Menge und können durch Kristallisation oder chromatographische Trennung der Ester an Kieselgel gewonnen werden.

Cis, endo-2-Azabicyclo-[5.3.0]-decan-3-carbonsäureester der Formel IIIa und b sind aus Enaminen des Cycloheptanons mit der Formel VI, in welcher $X^1$ für Dialkylamino mit 2 bis 10 C-Atomen oder für einen Rest der Formel VII, worin m und o eine ganze Zahl von 1 bis 3, $(m + o) \geq 3$ und A $CH_2$, NH, O oder S bedeutet, steht,

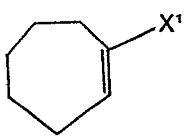
(VI)

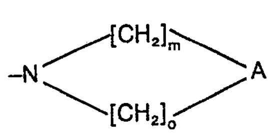
(VII)

und N-acylierten β-Halogen-α-amino-carbonsäureestern der Formel VIII, in welcher $X^2$ für eine nucleofuge Gruppe, vorzugsweise Chlor oder Brom, $Y^1$ für Alkanoyl mit 1 bis 5 C-Atomen, Aroyl mit 7 bis 9 C-Atomen oder andere, in der Peptidchemie übliche, sauer abspaltbare Schutzgruppen und $R^2$ für Alkyl mit 1 bis 5 C-Atomen oder Aralkyl mit 7 bis 9 C-Atomen steht

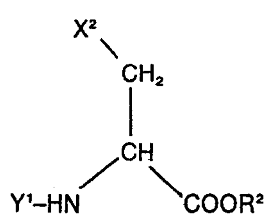
(VIII)

oder mit Acrylsäureestern der Formel IX, in welcher $Y^1$ und $R^2$ vorstehende Bedeutung haben, zugänglich,

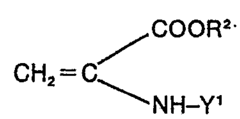
(IX)

indem man diese zu Verbindungen der Formel X, in welcher $R^2$ und $Y^1$ vorstehende Bedeutung haben, umsetzt

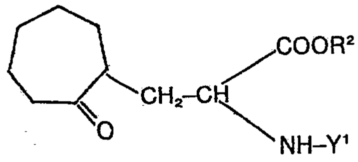

(X)

diese mit Hilfe starker Säuren unter Acylamid- und Esterspaltung zu Verbindungen der Formel XIa oder b cyclisiert,

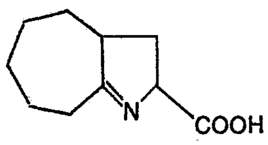

a

(XI)

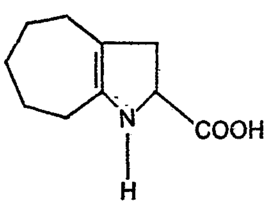

b

diese durch katalytische Hydrierung in Gegenwart von Übergangsmetallkatalysatoren oder durch Reduktion mit Boran-Aminkomplexen oder komplexen Borhydriden in niederen Alkoholen in Verbindungen der Formeln IIIa und/oder b, in welcher W für Wasserstoff steht, überführt und gegebenenfalls zu Verbindungen der Formel IIIa und/oder b, in welcher W für Alkyl mit 1 bis 6 C-Atomen oder Aralkyl mit 7 bis 8 C-Atomen steht, verestert.

Racemische Gemische, bestehend aus Verbindungen der Formeln IIIa und IIIb, können, falls es gewünscht wird, nach den bekannten Methoden der Racematspaltung (vgl. z.B. Quart. Rev. 25 (1971) 323 ff.) voneinander getrennt werden.

Die bicyclischen Aminosäuren der Formeln IIIa und b besitzen die cis, endo-Konfiguration, d.h. die $-CO_2W$-Gruppe ist dem Cycloheptanring zugewandt. Auch alle weiteren in der vorliegenden Erfindung aufgeführten 2-Azabicyclo-[5.3.0]-decan-3-carbonsäure-Derivate liegen in der cis, endo-Konfiguration vor.

Bevorzugte Enamine sind beispielsweise Pyrrolidinocyclohepten und Morpholinocyclohepten. Die Cyclisierung der Alkylierungsprodukte der Formel X erfolgt vorzugsweise mit wässriger Chlorwasserstoffsäure. Die Verbindungen der Formel IIIa und b (mit W = H) oder deren racemisches Gemisch können nach den bei Aminosäuren üblichen Methoden (s. z.B. Houben-Weyl, Methoden der organischen Chemie, Bd. VIII (1952) verestert werden, z.B. mit Thionylchlorid/Benzylalkohol oder Isobutylen/Schwefelsäure. Nach entsprechender Aufarbeitung erhält man Verbindungen der Formel IIIa und/oder b in Form der freien Base oder eines Salzes.

Die neuen Verbindungen der Formel I besitzen eine langdauernde, intensive blutdrucksenkende Wirkung. Sie sind starke Hemmer des Angiotensin-Converting-Enzyms (ACE-Hemmer) und können zur Bekämpfung des Bluthochdrucks verschiedener Genese eingesetzt werden. Auch ihre Kombination mit anderen blutdrucksenkenden, gefässerweiternden oder diuretisch wirksamen Verbindungen sind möglich. Typische Vertreter dieser Wirkklassen sind z.B. in Erhardt-Ruschig, Arzneimittel, 2. Auflage, Weinheim, 1972, beschrieben. Die Anwendung kann intravenös, subcutan oder peroral erfolgen.

Die Dosierung bei oraler Gabe liegt bei 1–100 mg, vorzugsweise 1–50, insbesondere 1–30 mg je Einzeldosis für einen normalgewichtigen Erwachsenen; dies entspricht 0,013 bis 1,3 mg/kg/Tag, vorzugsweise 0,013 bis 0,7 mg/kg/Tag, insbesondere 0,013 bis 0,4 mg/kg/Tag. Sie kann in schweren Fällen auch erhöht werden, da toxische Eigenschaften bisher nicht beobachtet wurden. Auch eine Herabsetzung der Dosis ist möglich und vor allem dann angebracht, wenn gleichzeitig Diuretika verabreicht werden.

Die erfindungsgemässen Verbindungen können oral oder parenteral in entsprechender pharmazeutischer Zubereitung verabreicht werden. Für eine orale Anwendungsform werden die aktiven Verbindungen mit den dafür üblichen Zusatzstoffen wie Trägerstoffen, Stabilisatoren oder inerten Verdünnungsmitteln vermischt und durch übliche Methoden in geeignete Darreichungsformen gebracht, wie Tabletten, Dragees, Steckkapseln, wässrige alkoholische oder ölige Suspensionen oder wässrige alkoholische oder ölige Lösungen. Als inerte Träger können z.B. Gummi arabicum, Magnesiumcarbonat, Kaliumphosphat, Milchzukker, Glucose oder Stärke, insbesondere Maisstärke verwendet werden. Dabei kann die Zubereitung sowohl als Trocken- oder Feuchtgranulat erfolgen. Als ölige Trägerstoffe oder Lösungsmittel kommen beispielsweise pflanzliche und tierische Öle in Betracht, wie Sonnenblumenöl oder Lebertran.

Zur subkutanen oder intravenösen Applikation werden die aktiven Verbindungen oder deren physiologisch verträgliche Salze, gewünschtenfalls mit den dafür üblichen Substanzen wie Lösungsvermittler, Emulgatoren oder weitere Hilfsstoffe in Lösung, Suspension oder Emulsion gebracht. Als Lösungsmittel für die neuen aktiven Verbindungen und die entsprechenden physiologisch verträglichen Salze kommen z.B. in Frage: Wasser, physiologische Kochsalzlösungen oder Alkohole, z.B. Ethanol, Propandiol oder Glycerin, daneben auch Zuckerlösungen wie Glucose- oder Mannitlösungen, oder auch eine Mischung aus den verschiedenen genannten Lösungsmitteln.

Die nachfolgenden Beispiele sollen die erfindungsgemässen Verfahrensweisen erläutern, ohne die Erfindung auf die hier stellvertretend genannten Substanzen zu beschränken.

Die $^1H$-NMR-Daten beziehen sich, wenn nicht anders angegeben, auf $CDCl_3$-Lösungen und sind δ-Werte.

Beispiel I:
N-(1-S-Carbethoxy-3-phenyl-propyl)-S-alanyl-cis, endo-2-azabicyclo-[5.3.0]-decan-3-S-carbonsäure

(1) 2-Acetylamino-3-(2-oxo-cycloheptyl)-propionsäure-methylester

25,7 g 3-Chlor-2-acetylamino-propionsäuremethylester und 30 g Cycloheptenopyrrolidin werden in 170 ml DMF 36 Stunden bei Raumtemperatur gehalten. Man engt im Vakuum ein, nimmt den Rückstand in wenig Wasser auf, stellt mit konzentrierter Salzsäure auf pH 2 und extrahiert 2 mal mit je 200 ml Essigester. Beim Einengen der organischen Phase hinterbleibt ein hellgelbes Öl.

Ausbeute: 44 g

$^1$H-NMR: 2,1 (s, 3H); 3,7 (s, 3H); 4,4–4,8 (m, 1H)

(2) cis, endo-2-Azabicyclo-[5.3.0]-decan-3-carbonsäure-hydrochlorid

44 g des unter (1) hergestellten Acetylamino-Derivates werden in 250 ml 2 n Salzsäure 90 Minuten am Rückfluss gekocht. Man engt im Vakuum ein, nimmt den Rückstand in Eisessig auf, versetzt mit 2 g Pt/C (10% Pt) und hydriert. Nach Filtration wird eingeengt und der Rückstand aus Essigester/Diisopropylether kristallisiert.

Schmelzpunkt: 252–256 °C (enthält laut NMR noch Essigsäure)

Ausbeute: 20 g

(3) cis, endo-2-Azabicyclo-[5.3.0]-decan-3-carbonsäure-benzylester-hydrochlorid

7,7 g der unter (2) hergestellten Carbonsäure werden in eine eiskalte Mischung aus 70 ml Benzylalkohol und 7,1 ml Thionylchlorid gegeben und 48 Stunden bei 5 °C belassen. Nach Einengen im Vakuum kristallisieren 7,3 g des Benzylesters aus Diisopropyläther. $^1$H-NMR (der Base in $CDCl_3$, 400 MHz) 1,1–2,0 (m, 11H), 2,1 (s, 1H), 2,2–2,4 (m, 2H); 3,3 (m, 1H); 3,3 (m, 1H); 3,7 (m, 1H); 5,2 (dd, 2H); 7,3 (s, 5H).

(4) N-(1-S-Carbethoxy-3-phenyl-propyl)-S-alanyl-cis,endo-2-azabicyclo-[5.3.0]-decan-3-S-carbonsäurebenzylester

3,5 g des nach (3) hergestellten Benzylesters werden mit 1,7 g HOBt, 3,6 g N-(1-S-Carbethoxy-3-phenyl-propyl)-S-alanin, 2,7 g Dicyclohexylcarbodiimid und 1,6 ml N-Ethyl-morpholin in 15 ml Dimethylformamid zur Reaktion gebracht. Nach 10 Stunden Rühren bei Raumtemperatur saugt man von ausgefallenem Dicyclohexylharnstoff ab, engt ein, nimmt in Methylenchlorid auf und schüttelt 2 × mit gesättigter $NaHCO_3$-Lösung aus. Die organische Phase wird nach dem Trocknen eingeengt und das erhaltene Rohprodukt (6,3 g) mit Cyclohexan/Essigester im Verhältnis 2:8 über eine Säule aus Kieselgel chromatographiert. Das zuerst eluierte Isomere stellt die S,S,S-Verbindung dar, ein späteres Eluat liefert nach dem Einengen die S,S,R-Verbindung.

$R_f$ der S,S,S-Verbindung: 0,46 ($SiO_2$; Cyclohexan/Essigester 1:4)

$R_f$ der S,S,R-Verbindung: 0,38

(5) N-(1-S-Carbethoxy-3-phenyl-propyl)-S-alanyl-cis,endo-2-azabicyclo-[5.3.0]-decan-3-S-carbon-säurehydrochlorid

0,5 g des S,S,S-Benzylesters aus (4) werden in 15 ml Ethanol gelöst und unter Zusatz von 0,1 g 10% Pd/C bei Normaldruck hydrogenolytisch entbenzyliert. Nach Aufnahme der berechneten Menge Wasserstoff wird vom Katalysator abfiltriert und im Vakuum eingeengt.

Ausbeute: 0,45 g Öl. Es wird in Ethanol gelöst und mit ethanolischer HCl auf pH 4,5 eingestellt. Es wird eingeengt und der Rückstand mit Diisopropylester verrieben. Fp: ab 124 °C Zersetzung. Durch Zugabe von wässrigen Zinksalzen zu einer konzentrierten methanolischen Lösung der Titelverbindung (Zwitterion) kann ein thermisch besonders stabiles Zink-Komplexsalz erhalten werden.

$^1$H–NMR: 0,9–3,1 (m, 24H); 3,2–4,9 (m, 5H); 7,2 (s, 5H)

(6) N-(1-S-Carbethoxy-3-phenyl-propyl)-S-alanyl-cis,endo-2-azabicyclo-[5.3.0]-decan-3-R-carbon-säurehydrochlorid

Die Verbindung wird analog dem Verfahren des Beispiels I (5) aus dem S,S,R-Benzylester aus Beispiel I (4) hergestellt.

$^1$H–NMR: 1,0–3,1 (m, 24H); 3,3–4,9 (m, 5H); 7,2 (s, 5H).

Beispiel II:

(1) cis, endo-2-azabicyclo-[5.3.0]-decan-3-carbonsäure-tert.butylester

2,5 g 2-Azabicyclo-[5.3.0]-decan-3-carbonsäure-hydrochlorid aus Beispiel I (2) werden in 25 ml Dioxan mit 25 ml Isobutylen und 2,5 ml konzentrierter Schwefelsäure zur Reaktion gebracht. Nach 10 Stunden bei Raumtemperatur wird mit Natronlauge alkalisch gestellt, im Vakuum eingeengt, mit 100 ml Wasser versetzt und der Ester ausgeethert. Nach Abdampfen des Ethers erhält man 2 g farbloses Öl.

$^1$H–NMR: 1,1–2,0 (m, 11H); 1,2 (s, 9H); 2,1 (s, NH); 2,2–2,5 (m, 2H); 3,2–3,4 (m, 1H); 3,6–3,8 (m, 1H).

(2) N-(1-S-Carbobenzyloxy-3-oxo-3-phenyl-propyl)-S-alanin-tert.-butylester

12,0 g Acetophenon, 17 g Glyoxysäurebenzylester und 31,7 g S-Alanin-tert.butylester-toluolsulfonat werden in 200 ml Eisessig auf 45–50 °C 24 bis 48 Stunden erhitzt. Die Reaktion wird dünnschichtchromatographisch verfolgt und am optimalen Umsetzungspunkt abgebrochen. Man engt im Vakuum gut ein, stellt mit wässriger Bicarbonatlösung basisch und extrahiert mit Essigester. Man engt die organische Phase möglichst weitgehend ein und kristallisiert das S,S-Isomere aus Cyclohexan/Petrolether. Die R,S-Verbindung bleibt weitgehend in Lösung. Zum Erhalt von Impfkristallen empfiehlt sich eine Chromatographie des Rohgemisches an Kieselgel im System Cyclohexan/Essigester 2:1 dem man 0,1% Triäthylamin zusetzt. Die S,S-Verbindung wird als zweite der beiden Diastereomeren eluiert.

Man erhält 9 g.

| Analyse: | | C | H | N |
|---|---|---|---|---|
| $C_{24}H_{29}NO_5$: | ber. | 70,1 | 7,1 | 3,4 |
| | gef. | 70,0 | 6,9 | 3,5 |

(3) N-(1-S-Carbobenzyloxy-3-oxo-3-phenyl-propyl)-S-alanin-trifluoracetat

8 g des Mannich-Kondensationsproduktes aus (2) werden in 25 ml wasserfreier Trifluoressigsäure gelöst und eine Stunde bei Raumtemperatur belassen. Man engt im Vakuum ein, versetzt mit Diisopropylether und fällt mit Petrolether, man erhält 7,2 g amorphe Substanz.

| Analyse: | | C | H | N |
|---|---|---|---|---|
| $C_{22}H_{22}NO_7F_3$: | ber. | 56,3 | 4,7 | 3,0 |
| | gef. | 56,0 | 4,8 | 3,1 |

MW: 469

(4) N-(1-S-Carbobenzyloxy-3-oxo-3-phenyl-propyl)-S-alanyl-cis, endo-2-azabicyclo-[5.3.0]-decan-3-carbonsäure-tert.-butylester

3,5 g des N-substituierten Alanins aus (3) reagiert mit 2,1 g cis, endo-2-Azabicyclo-[5.3.0]-decan-3-carbonsäure-tert.-butylester aus Beispiel II (1) analog Beispiel I (4). Man erhält nach Chromatographie über Kieselgel 2 g der Titelverbindung.

(5) N-(1-S-Carbobenzyloxy-3-oxo-3-phenyl-propyl)-S-alanyl-cis,endo-2-azabicyclo-[5.3.0]-decan-3-carbonsäure

2 g des tert. Butylesters aus (4) werden in 50 ml Trifluoressigsäure gelöst und eine Stunde bei Raumtemperatur belassen.

Man engt im Vakuum ein, nimmt das zurückbleibende Harz in Essigester auf und neutralisiert mit wässrigem Bicarbonat. Aus der Essigesterphase werden 1,4 g der Titelverbindung gewonnen.

| Analyse: | | C | H | N |
|---|---|---|---|---|
| $C_{30}H_{36}N_2O_6$: | ber. | 69,2 | 7,0 | 5,4 |
| | gef. | 68,9 | 7,1 | 5,2 |

(6) N-(1-S-Carboxy-3-R,S-hydroxy-3-phenyl-propyl)-S-alanyl-cis, endo-2-azabicyclo-[5.3.0]-decan-3-carbonsäure

1 g N-(1-S-Carbobenzyloxy-3-oxo-3-phenyl-propyl)-S-alanyl-cis, endo-2-azabicyclo-[5.3.0]-decan-3-carbonsäure werden in 50 ml Ethanol gelöst, mit 150 mg Pd/$BaSO_4$ versetzt und bei Normaldruck hydriert. Nach Aufnahme der berechneten Wasserstoffmenge wird filtriert, eingeengt und über Kieselgel im Solvens $CHCl_3/CH_3OH/CO_2H$ 50:20:5 chromatographiert.

Ausbeute: 0,6 g

(7) N-(1-S-Carbobenzyloxy-3-R,S-hydroxy-3-phenyl-propyl)-S-alanyl-cis, endo-2-azabicyclo-[5.3.0]-decan-3-carbonsäure

1 g N-(1-S-Carbobenzyloxy-3-oxo-3-phenyl-propyl)-S-alanyl-cis,endo-2-azabicyclo-[5.3.0]-decan-3-carbonsäure werden in 50 ml eines Gemisches aus Acetonitril und Wasser gelöst und mit 150 mg $NaBH_4$ reduziert. Nach 12 Stunden wird zur Trokkene eingeengt, mit verdünnter Salzsäure neutral gestellt und die Titelverbindung mit Essigester extrahiert. Zur Entfernung von Borsäure und anderen Verunreinigungen wird über Kieselgel im Solvens $CHCl_3–CH_3OH–CH_3COOH$ 50:10:5 chromatographiert.

Beispiel III

Allgemeine Methode: Esterverseifung zur Darstellung von Verbindungen der Formel I mit $R^2 = H$.

1 g des entsprechenden Ethyl oder Benzylesters der Formel I (R = H) werden in 200 ml Dimethoxyethan gelöst. Man fügt einen Tropfen einer verdünnten Indikatorlösung, z. B. Bromthymolblau, zu und fügt unter starkem Rühren im Verlauf von 5 Minuten eine äquivalente Menge 4n KOH (wässrig) hinzu, so dass der Indikator bei Beendigung der Reaktion einen pH-Wert von 9–10 anzeigt. Sodann stellt man mit Salzsäure auf pH 4, engt im Vakuum zur Trockne ein, nimmt in 25 ml Essigester auf und filtriert. Beim Einengen des Essigesters fallen die Dicarbonsäuren als feste, kristalline oder amorphe Verbindungen an.

Die Ausbeuten liegen zwischen 60 und 95%

Beispiel IIIa

N-(1-S-Carboxy-3-phenyl-propyl)-S-alanyl-cis, endo-2-azabicyclo-[5.3.0]-decan-3-S-carbonsäure

1 g N-(1-S-Carbethoxy-3-phenyl-propyl)-S-alanyl-cis,endo-2-azabicyclo-[5.3.0]-decan-3-S-carbonsäure aus Beispiel I (5) wird wie unter Beispiel III beschrieben verseift (1 Stunde) und aufgearbeitet.

Ausbeute: 0,85 g

m/e: 416

Beispiel IV

N-(1-S-Carbethoxy-3-oxo-3-phenyl-propyl)-S-alanin-benzyl-ester

65,7 g 4-Phenyl-4-oxo-buten-2-carbonsäure-ethylester (Benzoylacrylsäureethylester) werden in 225 ml Ethanol gelöst und dazu 1 ml Triethylamin gegeben. Zu dieser Lösung wird bei Raumtemperatur eine Lösung von 70 g S-Alaninbenzylester in 90 ml Ethanol rasch zugetropft. Es wird 2 Stunden bei Raumtemperatur gerührt und dann die Lösung abgekühlt.

Es kristallisiert das S,S-Isomere aus.

Ausbeute: 94,3 g Fp.: 73–74 °C

$$[\alpha]_D^{20} = + 17,8\,°C \ (c = 1, CH_3OH)$$

Beispiel V

N-(1-S-Carbethoxy-3-oxo-3-phenyl-propyl)-S-alanin

0,5 g der Verbindung aus Beispiel IV werden in 40 ml Ethanol gelöst und 0,1 g Pd/C 10%ig zugegeben und bei Raumtemperatur und Normaldruck hydriert.

Ausbeute: 300 mg Fp.: 210–220 °C

$^1H$-NMR (DMSO-$d_6$): 1,0–1,4 (t, 6H); 3,2–5,0 (m, 8H); 7,2–8,2 (m, 5H)

Beispiel VI

N-(1-S-Carbethoxy-3-oxo-3-phenyl-propyl)-S-alanyl-cis,endo-2-azabicyclo-[5.3.0]-decan-3-S-carbonsäure-benzylester

Die Verbindung wird aus cis,endo-2-Azabicyclo-[5.3.0]-decan-3-S-carbonsäurebenzylester-hydrochlorid und N-(1-S-Carbethoxy-3-oxo-3-phenyl-propyl)-S-alanin aus Beispiel V analog dem Verfahren, das im Beispiel I (4) beschrieben ist, hergestellt.

Beispiel VII
N-(1-S-Carbethoxy-3-oxo-3-phenyl-propyl)-S-alanyl-cis,endo-2-azabicyclo-[5.3.0]-decan-3-S-carbonsäure

1 g des Benzylesters aus Beispiel VI werden in 30 ml Ethanol gelöst, mit 100 mg Pd/C (10%ig) bei Raumtemperatur und Normaldruck hydriert. Nach der Aufnahme eines Moläquivalentes Wasserstoff wird die Hydrierung abgebrochen. Es wird vom Katalysator abgesaugt, die Lösung eingeengt.

Ausbeute: 600 mg Öl.

$^1$H–NMR (DMSO-$d_6$): 1,0–3,0 (m, 19H); 3,2–4,9 (m, 10H); 7,2–8,1 (m, 5H)

Beispiel VIII
$N_\alpha$-(1-S-Carbethoxy-3-phenyl-propyl)-S-lysyl-cis,endo-2-azabicyclo-[5.3.0]-decan-3-S-carbonsäure-dihydrochlorid

(1) $N_\alpha$-(1-S-Carbethoxy-3-oxo-3-phenyl-propyl)-$N_\varepsilon$-benzyl-oxycarbonyl-S-lysinbenzylester

10 g 4-Phenyl-4-oxo-buten-2-carbonsäureethylester werden in 100 ml Ethanol gelöst. Dazu werden 19,1 g $N_\varepsilon$-benzyl-oxycarbonyl-S-lysinbenzylester und 0,2 g Triethylamin gegeben. Die Lösung wird 3 Stunden bei Raumtemperatur gerührt, danach im Vakuum eingeengt. Der ölige Rückstand (31 g) wird in Isopropanol/Diisopropylether gelöst und abgekühlt. Es kristallisieren 13 g $N_\alpha$-(1-S-Carbethoxy-3-oxo-3-phenyl-propyl)-$N_\varepsilon$-benzyloxycarbonyl-S-lysinbenzylester.

$\alpha_D^{20} = 3,5°$ (c = 1, $CH_3OH$)

$^1$H–NMR ($CDCl_3$): 1,0–1,4 (tr, 3H); 1,0–2,0 (m, 9H); 2,0–2,6 (breites s., 1H); 2,9–3,9 (m, 6H); 3,9–4,4 (q, 2H); 4,6–4,9 (breites s, 1H); 5,0–5,2 (doppeltes s, 4H) 7,1–8,1 (m, 15H)

(2) $N_\alpha$-(1-S-Carbethoxy-3-phenyl-propyl)-$N_\varepsilon$-benzyloxy-carbonyl-S-lysin

4,0 g des in Beispiel VIII (1) hergestellten Lysinbenzylesterderivates werden in 50 ml Eisessig gelöst, dazu 0,6 g Pd/C (10%ig) und 0,6 g konz. Schwefelsäure gegeben. Es wird 6 Stunden bei Raumtemperatur und unter Normaldruck hydriert. Danach wird vom Katalysator abgesaugt, die ethanolische Lösung mit 1,4 g festem Natriumhydrogencarbonat gerührt. Die Lösung wird einrotiert und der Rückstand in Wasser gelöst. Die wässrige Phase wird mit Essigester und Methylenchlorid extrahiert. Die organischen Phasen werden verworfen und die wässrige Phase im Vakuum zur Trockne eingedampft. Der Rückstand wird mit Methanol ausgerührt. Nach dem Abdampfen des Methanol bleibt ein öliger Rückstand, der bei Behandlung mit Diisopropylether fest wird. Ausbeute an $N_\alpha$-(1-S-Carbethoxy-3-phenyl-propyl)-S-lysin: 2,0 g.

$^1$H–NMR ($D_2O$): 1,0–1,4 (tr, 3H); 1,0–2,5 (m, 9H); 2,5–4,4 (m, 9H); 3,9–4,4 (q, 2H); 4,5–5,0 (m, 1H); 7,1–7,6 (m, 5H)

m/e: 336

3,4 g $N_\alpha$-(1-S-Carbethoxy-3-phenyl-propyl)-S-lysin werden in 30 ml Methylenchlorid gelöst und auf 0 °C abgekühlt. Dazu werden unter Eiskühlung 2,1 g Triethylamin gegeben und anschliessend 1,9 g Chlorameisenbenzylester zugetropft. Es wird 1 Stunde bei 0 °C gerührt und dann auf Raumtemperatur gebracht. Die Methylenchloridlösung wird dann mit Wasser, Natriumcarbonatlösung und Wasser ausgeschüttelt. Nach dem Trocknen wird eingeengt und der ölige Rückstand über Kieselgel mit Methylenchlorid/Methanol chromatographiert.

Es werden 2,0 g $N_\alpha$-(1-S-Carbethoxy-3-phenylpropyl)-$N_\varepsilon$-benzyloxycarbonyl-S-lysin erhalten.

$^1$H–NMR ($CDCl_3/D_2O$): 1,0–1,4 (tr, 3H); 1,0–2,5 (m, 9H); 2,5–4,4 (m, 9H); 3,9–4,4 (q, 2H); 4,4–5,0 (m, 1H); 5,1 (s, 2H); 7,1–7,5 (m, 10H).

(3) $N_\alpha$-(1-S-Carbethoxy-3-phenyl-propyl)-$N_\varepsilon$-benzyloxycarbonyl-S-lysyl-cis,endo-2-azabicyclo-[5.3.0]-decan-3-S-carbonsäurebenzylester

a) 560 mg 2-Azabicyclo-[5.3.0]-decan-3-carbonsäure-benzylester-hydrochlorid, hergestellt nach Beispiel I (3), werden mit 940 mg $N_\alpha$-(1-S-Carbethoxy-3-phenyl-propyl)-$N_\varepsilon$-benzyloxycarbonyl-S-lysin, hergestellt nach Beispiel VIII (2), analog Beispiel I (4) umgesetzt. Man erhält nach der Aufarbeitung 1,5 g Öl, das ein Gemisch zweier diastereomerer Verbindungen ist.

Das Diastereomerengemisch wird säulenchromatographisch mit Kieselgel und Cyclohexan/Essigester 2:1 als Elutionsmittel in die Einzelkomponenten getrennt. Das zuerst eluierte Isomere stellt obige Verbindung dar. Es werden 0,6 g Öl erhalten.

$^1$H-NMR ($CDCl_3$): 1,0–2,6 (m, 24H); 2,6–4,5 (m, 8H);

(nach H/D-Austausch mit $D_2O$): 4,6–5,0 (m, 2H); 5,1–5,3 (doppeltes s, 4H); 7,1–7,6 (m, 15H)

b) Das spätere Eluat liefert 0,4 g N-(1-S-Carbethoxy-3-phenyl-propyl)-$N_\varepsilon$-benzyloxycarbonyl-S-lysyl-cis,endo-2-azabicyclo-[5.3.0]-decan-3-R-carbonsäurebenzylester.

$^1$H-NMR ($CDCl_3$): 1,0–2,6 (m, 24H); 2,6–4,4 (m, 8H);

(nach H/D-Austausch mit $D_2O$): 4,5–5,0 (m, 2H); 5,1–5,3 (doppeltes s, 4H); 7,1–7,5 (m, 15H)

(4) $N_\alpha$-(1-Carbethoxy-3-phenyl-propyl)-S-lysyl-cis,endo-2-azabicyclo-[5.3.0]-decan-3-S-carbon-säure-dihydrochlorid

500 mg $N_\alpha$-(1-S-Carbethoxy-3-phenyl-propyl)-$N_\varepsilon$-benzyloxycarbonyl-S-lysyl-cis,endo-2-azabicyclo-[5.3.0]-decan-3-S-carbonsäurebenzylester aus Beispiel VIII (3a) werden in 20 ml Ethanol gelöst und unter Zusatz von 0,1 g 10%ig Pd/C bei Normaldruck hydrogenolytisch entbenzyliert. Nach Beendigung der Wasserstoffaufnahme wird vom Katalysator abfiltriert, die ethanolische Lösung mit ethanolischer Chlorwasserstofflösung bis pH 1 versetzt, das Ethanol im Vakuum abgedampft. Der Rückstand wird mit Diisopropylether versetzt, wobei das Produkt fest wird. Es werden 200 mg erhalten.

$^{1}$N–NMR des Betains: 1,0–2,5 (m, 24H); 2,6–4,4 (m, 8H);
($CDCl_3$, nach H/D-Austausch mit $D_2O$): 4,4–4,9 (m, 2H); 7,2 (s, 5H)

Beispiel IX
$N_\alpha$-(1-S-Carbethoxy-3-phenyl-propyl)-S-lysyl-cis,endo-2-azabicyclo-[5.3.0]-decan-3-R-carbonsäure-dihydrochlorid

0,3 mg des entsprechenden Benzylesters aus Beispiel VIII (3b) werden analog Beispiel VIII (4) umgesetzt und aufgearbeitet. Es werden 130 mg der Carbonsäure als Dihydrochlorid erhalten.

$^{1}$H-NMR des Betains ($CDCl_3$, nach H/D-Austausch mit $D_2O$): 1,0–2,6 (m, 24H); 2,6–4,4 (m, 8H); 4,4–4,8 (m, 2H); 7,2 (s, 5H)

Beispiel X
$N_\alpha$-(1-S-Carboxy-3-phenyl-propyl)-S-lysyl-cis, endo-2-azabicyclo-[5.3.0]-decan-3-S-carbonsäure-hydrochlorid

0,5 g $N_\alpha$-(1-S-Carbethoxy-3-phenyl-propyl)-S-lysyl-cis,endo-2-azabicyclo-[5.3.0]-decan-3-S-carbonsäuredihydrochlorid aus Beispiel VIII (4) werden in 20 ml Dimethoxyethan suspendiert. Es wird eine halbe Stunde gerührt. Danach stellt man mit Salzsäure auf pH 4 ein, engt im Vakuum zur Trockne ein, nimmt den Rückstand in Essigester auf und filtriert. Die Essigesterlösung wird eingeengt, der Rückstand wird mit Diisopropylester verrieben, wobei er fest wird.

Ausbeute: 0,30 g

$^{1}$H–NMR ($D_2O$): 1,2–2,5 (m, 21H); 2,5–4,5 (m, 6H); 4,5–4,9 (m, 2H); 7,2 (s, %H)

Beispiel XI
$N_\alpha$-(1-S-Carboxy-3-phenyl-propyl)-S-lysyl-cis, endo-2-azabicyclo-[5.3.0]-decan-3-R-carbonsäure-hydrochlorid

500 mg $N_\alpha$-(1-S-Carbethoxy-3-phenyl-propyl)-S-lysyl-cis,endo-2-azabicyclo-[5.3.0]-decan-3-R-carbonsäure-dihydrochlorid aus Beispiel IX werden analog Beispiel X verseift und aufgearbeitet.

Ausbeute: 0,30 g

$^{1}$H–NMR ($D_2O$): 1,2–2,5 (m, 21H); 2,5–4,5 (m, 6H); 4,5–4,9 (m, 2H); 7,2 (s, 5H)

Beispiel XII
N-(1-S-Carbethoxy-3-phenyl-propyl)-O-ethyl-S-tyrosyl-cis,endo-2-azabicyclo-[5.3.0]-decan-3-S-carbonsäure

(1) N-(1-R,S-Carbethoxy-3-phenyl-propyl)-O-ethyl-S-tyrosin-benzylester

Man setzt analog Beispiel IV 24 g Benzoylacrylsäureethylester in 100 ml Ethanol mit 30 g O-Ethyl-S-tyrosinbenzylester in Anwesenheit von 0,5 ml Triethylamin um und erhält nach Einengen der Lösung und Digerieren des Rückstandes mit Diethylester/Petrolether (1:1) und Trocknen im Vakuum 42 g der RS,S-Verbindung.

(2) N-(1-R,S-Carbethoxy-3-phenyl-propyl)-O-ethyl-S-tyrosin

40 g der nach XII (1) erhaltenen Verbindung werden in 800 ml Essigsäure mit 4 g Pd/C (10%) bei 100 bar und Raumtemperatur hydriert. Ausb. nach Chromatographie an Kieselgel im Elutionsmittel Essigester/Cyclohexan (1:3) und Trocknen des Eindampfrückstandes 25 g dünnschichtchromatographisch nahezu einheitliche Titelverbindung.

Schm. 205–213°

$C_{23}H_{29}NO_5$(399,5)

| | | | |
|---|---|---|---|
| Ber. | C 69,15 | H 7,31 | N 3,50 |
| Gef. | C 69,5 | H 7,4 | N 3,3 |

(3) N-(1-S-Carbethoxy-3-phenyl-propyl)-O-ethyl-S-tyrosyl-cis,endo-2-azabicyclo-[5.3.0]-decan-3-S-carbonsäure

Man setzt analog Beispiel I (4) 1 g des nach Beispiel I (3) und Ausschütteln mit Diethylether aus alkalischer Lösung erhaltenen freien Benzylesters mit 1,6 g der nach XII (2) erhaltenen Verbindung mittels 0,9 g Dicyclohexylcarbodiimid in Anwesenheit von 0,55 g 1-Hydroxybenzotriazol um. Nach der unter Beispiel I (4) beschriebenen Chromatographie erhält man 0,7 g öligen Benzylester als Zwischenprodukt.

Die $^{1}$H–NMR- und Massenspektren sind im Einklang mit der angegebenen Struktur.

Der Benzylester wird in 15 ml Ethanol unter Normaldruck an Pd(C) katalytisch hydriert. Nach Abfiltrieren des Katalysators und Abdestillieren des Lösungsmittels bleibt ein fester Rückstand zurück, der mit Diethylether/Petrolether digeriert und getrocknet wird.

Ausbeute: 0,4 g

$^{1}$H–NMR ($CDCl_3$): 1,2–3,0 (m, 19H); 1,27 (t, 3H); 1,4 (t, 3H); 3,0–4,3 (m, 4H); 3,8–4,2 (m, 4H); 6,5–7,1 (2d, 4H); 7,3 (s, 5H).

Beispiel XIII
N-(1-S-Carbethoxy-3-phenyl-propyl)-O-methyl-S-tyrosyl-cis,endo-2-azabicyclo-[5.3.0]-decan-3-S-carbonsäure

Man arbeitet wie in Beispiel XII beschrieben, setzt aber in der XII (1) analogen Stufe O-Methyl-S-tyrosin-benzyl-ester ein und erhält die Titelverbindung, deren $^{1}$H-NMR-Spektrum im Einklang mit der angegebenen Struktur ist.

**Patentansprüche für die Vertragsstaaten: BE, CH, DE, FR, GB, IT, LI, LU, NL, SE**

1. Verbindung der Formel I,

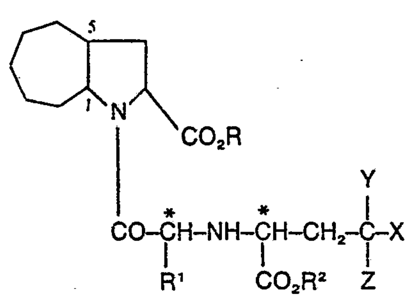

(I)

in der die Wasserstoffatome an den Brückenkopf-C-Atomen 1 und 5 zueinander cis-konfiguriert sind und die $CO_2R$-Gruppe am C-Atom 3 endoständig zum bicyclischen Ringsystem orientiert ist, in der

R = Wasserstoff, ($C_1$–$C_6$)-Alkyl, ($C_2$–$C_6$)-Alkenyl oder ($C_6$–$C_{12}$)-Aryl-($C_1$–$C_4$)-Alkyl,

$R^1$ = Wasserstoff, Allyl, Vinyl oder eine Seitenkette einer gegebenenfalls geschützten natürlich vorkommenden α-Aminosäure $R^1$–$CH(NH_2)$–COOH,

$R^2$ = Wasserstoff ($C_1$–$C_6$)-Alkyl, ($C_2$–$C_6$)-Alkenyl oder ($C_6$–$C_{12}$)-Aryl-($C_1$–$C_4$)-alkyl,

Y = Wasserstoff oder Hydroxy,

Z = Wasserstoff oder

Y und Z = zusammen Sauerstoff,

X = ($C_1$–$C_6$)-Alkyl, ($C_2$–$C_6$)-Alkenyl, ($C_5$–$C_9$)-Cycloalkyl, ($C_6$–$C_{12}$)-Aryl, das durch ($C_1$–$C_4$)-Alkyl, ($C_1$–$C_4$)-Alkoxy, Hydroxy, Halogen, Nitro, Amino, ($C_1$–$C_4$)-Alkylamino, Di-($C_1$–C4)-alkyl-amino und/oder Methylendioxy mono, di- oder trisubstituiert sein kann,

oder Indol-3-yl bedeuten,

sowie deren physiologisch unbedenklichen Salze.

2. Verbindung der Formel I gemäss Anspruch 1, in welcher

R = Wasserstoff

$R^1$ = Methyl, die gegebenenfalls acylierte Seitenkette von Lysin oder doe O–($C_1$–$C_6$)-alkylierte Seitenkette des Tyrosin

$R^2$ = Wasserstoff, Methyl, Ethyl, Benzyl oder tert. Butyl

X = Phenyl oder mit Fluor und/oder Chlor mono- oder disubstituiertes Phenyl,

Y = Wasserstoff oder Hydroxy,

Z = Wasserstoff oder

Y und Z zusammen Sauerstoff bedeuten.

3. 2-[N-(1-S-Carbethoxy-3-phenyl-propyl)-S-alanyl]-cis,endo-2-azabicyclo[5.3.0]decan-3-S-carbonsäure

sowie deren physiologisch unbedenklichen Salze.

4. 2-[N-(1-S-Carboxy-3-phenyl-propyl)-S-alanyl]-cis,endo-2-azabicyclo[5.3.0]decan-3-S-carbonsäure

sowie deren physiologisch unbedenklichen Salze.

5. 2-[Nα-(1-S-Carbethoxy-3-phenyl-propyl)-S-lysyl]-cis,endo-2-azabicyclo[5.3.0]decan-3-S-carbonsäure

sowie deren physiologisch unbedenklichen Salze.

6. 2-[Nα-(1-S-Carboxy-3-phenyl-propyl)-S-lysyl]-cis,endo-2-azabicyclo[5.3.0]decan-3-S-carbonsäure

sowie deren physiologisch unbedenklichen Salze.

7. 2-[N-(1-S-Carbethoxy-3-phenyl-propyl)-O-ethyl-S-tyrosyl]-cis,endo-2-azabicyclo-[5.3.0]decan-3-S-carbonsäure

sowie deren physiologisch unbedenklichen Salze.

8. 2-[N-(1-S-Carbethoxy-3-phenyl-propyl)-O-methyl-S-tyrosyl]-cis,endo-2-azabicyclo-[5.3.0]decan-3-S-carbonsäure

sowie deren physiologisch unbedenklichen Salze.

9. Verfahren zur Herstellung von Verbindungen gemäss Anspruch 1, dadurch gekennzeichnet, dass man eine Verbindung der Formel II

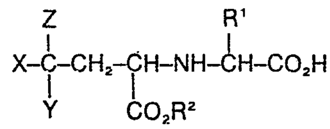

(II)

in der X, Y, Z, $R^1$ und $R^2$ die im Anspruch 1 definierten Bedeutungen mit Ausnahme von $R^2$ = Wasserstoff haben, mit einer Verbindung der Formeln IIIa oder IIIb oder dem Racemat

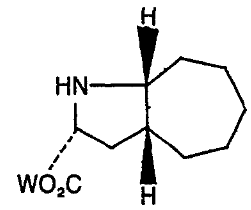

(IIIa)

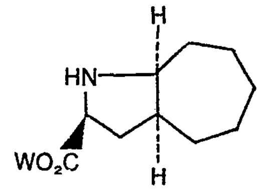

(IIIb)

in denen W Wasserstoff oder eine Carboxy veresternde Gruppe bedeutet, falls W Wasserstoff bedeutet, unter vorheriger Veresterung der Verbindung der Formel IIIa oder IIIb oder des Racemats zu Verbindungen der Formel I umsetzt, diese ggfs. durch Hydrierung, Säure- und/oder Basenbehandlung in Verbindung der Formel I (R und/oder $R^2$ = H) überführt, so erhaltene Verbindungen ggfs. in an sich bekannter Weise verestert und/oder ggfs. in ihre physiologisch unbedenklichen Salze überführt.

10. Mittel, bestehend aus oder enthaltend eine Verbindung gemäss einem der Ansprüche 1–8.

11. Verbindung der Formel I gemäss einem der Ansprüche 1 bis 8 zur Anwendung als Heilmittel.

12. Verbindung der Formel I gemäss einem der Ansprüche 1 bis 8 zur Anwendung als Heilmittel bei der Behandlung des Bluthochdrucks.

13. Verbindung der Formel I gemäss Anspruch 11 oder 12 zur Anwendung in Kombination mit einem Diuretikum.

14. Verbindung der Formeln IIIa und/oder IIIb, in welchen W Wasserstoff oder eine veresternde Gruppe, vorzugsweise Alkyl mit 1–6 C-Atomen oder Aralkyl mit 7 oder 8 C-Atomen bedeutet sowie deren Salze mit Säuren und (für den Fall W = Wasserstoff) mit Basen.

15. Verbindung gemäss Anspruch 14, dadurch gekennzeichnet, dass in Formeln IIIa und b W Wasserstoff, tert.-Butyl, Benzyl oder Nitrobenzyl bedeutet.

16. Verfahren zur Herstellung von Verbindungen der Formeln IIIa und/oder IIIb gemäss Anspruch 14 oder 15, dadurch gekennzeichnet, dass Enamine der Formel VI, in welcher $X^1$ für Dialkylamino mit 2 bis 10 C-Atomen oder für einen Rest der Formel VII, worin m und o eine ganze Zahl von 1 bis 3, (m + o) ⩾ 3 und A $CH_2$, NH, O oder S bedeuten, steht

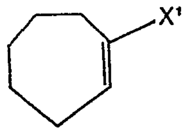

(VI)

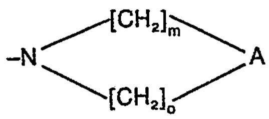

(VII)

mit N-acylierten β-Halogen-α-amino-carbonsäureestern der Formel VIII, in welcher $X^2$ für eine nucleofuge Gruppe, $Y^1$ für Alkanoyl mit 1 bis 5 C-Atomen, Aroyl mit 7 bis 9 C-Atomen oder andere, in der Peptidchemie übliche, sauer abspaltbare Schutzgruppen und $R^2$ für Alkyl mit 1 bis 5 C-Atomen oder Aralkyl mit 7 bis 9 C-Atomen steht

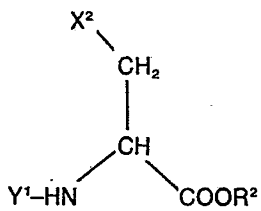

(VIII)

oder mit Acrylsäureestern der Formel IX, in welcher $Y^1$ und $R^2$ vorstehende Bedeutung haben

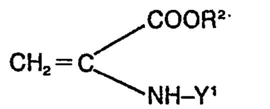

(IX)

zu Verbindungen der Formel X, in welcher $R^2$ und $Y^1$ vorstehende Bedeutung haben, umsetzt,

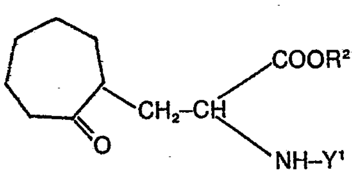

(X)

diese mit Hilfe starker Säuren unter Acylamid- und Esterspaltung zu Verbindungen der Formel XIa oder b cyclisiert,

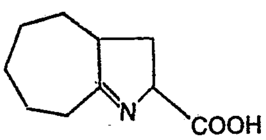

a

(XI)

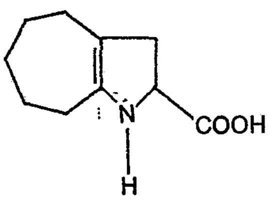

b

diese durch katalytische Hydrierung in Gegenwart von Übergangsmetallkatalysatoren oder durch Reduktion mit Boran-Aminkomplexen oder komplexen Borhydriden in niederen Alkoholen in Verbindungen der Formeln IIIa und/oder IIIb, in welchen W für Wasserstoff steht, überführt und gegebenenfalls zu Verbindungen der Formeln IIIa und/oder IIIb, in welchen W für eine veresternde Gruppe, vorzugsweise Alkyl mit 1 bis 6 C-Atomen oder Aralkyl mit 7 bis 8 C-Atomen steht, verestert und die so erhaltenen Verbindungen der Formel IIIa und/oder IIIb gegebenenfalls in ihre Salze überführt.

**Patentansprüche für den Vertragsstaat AT:**

1. Verfahren zur Herstellung von Verbindungen der Formel I

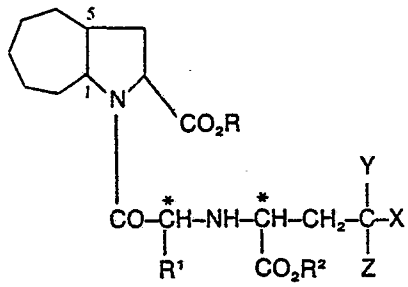

(I)

in der die Wasserstoffatome an den Brückenkopf-C-Atomen 1 und 5 zueinander cis-konfiguriert sind und die $CO_2R$-Gruppe am C-Atom 3 endoständig zum bicyclischen Ringsystem orientiert ist, in der

R = Wasserstoff, $(C_1$–$C_6)$-Alkyl, $(C_2$–$C_6)$-Alkylen oder $(C_6$–$C_{12})$-Aryl-$(C_1$–$C_4)$-Alkyl,

$R^1$ = Wasserstoff, Allyl, Vinyl oder eine Seitenkette einer gegebenenfalls geschützten natürlich vorkommenden α-Aminosäure $R^1$–$CH(NH_2)$–$COOH$,

$R^2$ = Wasserstoff $(C_1$–$C_6)$-Alkyl, $(C_2$–$C_6)$-Alkenyl oder $(C_6$–$C_{12})$-Aryl-$(C_1$–$C_4)$-Alkyl,

Y = Wasserstoff oder Hydroxy,

Z = Wasserstoff oder

Y und Z = zusammen Sauerstoff,

X = $(C_1$–$C_6)$-Alkyl, $(C_2$–$C_6)$-Alkenyl, $(C_5$–$C_9)$-Cycloalkyl, $(C_6$–$C_{12})$-Aryl, das durch $(C_1$–$C_4)$-Alkyl, $(C_1$–$C_4)$-Alkoxy, Hydroxy, Halogen, Nitro, Amino, $(C_1$–$C_4)$-Alkylamino, Di-$(C_1$–$C_4)$-alkyl-amino und/oder Methylendioxy mono-, di- oder trisubstituiert sein kann, oder Indol-3-yl bedeuten, sowie deren physiologisch unbedenklichen Salze, dadurch gekennzeichnet, dass man eine Verbindung der Formel II

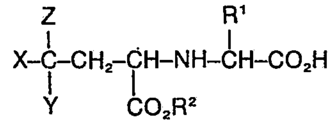

(II)

in der X, Y, Z, $R^1$ und $R^2$ die im Anspruch 1 definierten Bedeutungen mit Ausnahme von $R^2$ = Wasserstoff haben, mit einer Verbindung der Formeln IIIa oder IIIb oder dem Racemat

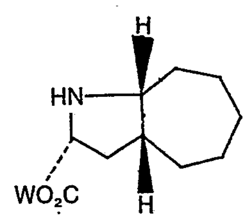

(IIIa)

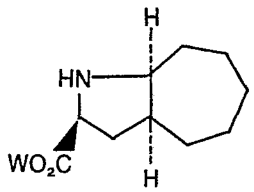

(IIIb)

in denen W Wasserstoff oder eine Carboxy veresternde Gruppe bedeutet, falls W Wasserstoff bedeutet, unter vorheriger Veresterung der Verbindung der Formel IIIa oder IIIb oder des Racemats zu Verbindungen der Formel I umsetzt, diese ggfs. durch Hydrierung, Säure- und/oder Basenbehandlung in Verbindungen der Formel I (R und/oder $R^2$ = H) überführt, so erhaltene Verbindungen ggfs. in an sich bekannter Weise verestert und/oder ggfs. in ihre physiologisch unbedenklichen Salze überführt.

2. Verfahren gemäss Anspruch 1 zur Herstellung einer Verbindung der Formel I, in welcher

R = Wasserstoff

$R^1$ = Methyl, die gegebenenfalls acylierte Seitenkette von Lysin oder die O–($C_1$–$C_6$)-alkylierte Seitenkette des Tyrosin

$R^2$ = Wasserstoff, Methyl, Ethyl, Benzyl oder tert. Butyl

X = Phenyl oder mit Fluor und/oder Chlor mono- oder disubstituiertes Phenyl,

Y = Wasserstoff oder Hydroxy

Z = Wasserstoff oder

Y und Z zusammen Sauerstoff bedeuten, sowie deren physiologisch unbedenklichen Salzen.

3. Verfahren gemäss Anspruch 1 oder 2 zur Herstellung von

2-[N-(1-S-Carbethoxy-3-phenyl-propyl)-S-alanyl]-cis,endo-2-azabicyclo[5.3.0]]decan-3-S-carbonsäure

oder dessen physiologisch unbedenklichem Salz.

4. Verfahren gemäss Anspruch 1 oder 2 zur Herstellung von

2-[N-(1-S-Carboxy-3-phenyl-propyl)-S-alanyl]-cis, endo-2-azabicyclo[5.3.0]decan-3-S-carbonsäure

oder dessen physiologisch unbedenklichem Salz.

5. Verfahren gemäss Anspruch 1 oder 2 zur Herstellung von

2-[Nα-(1-S-Carbethoxy-3-phenyl-propyl)-S-lysyl]-cis,endo-2-azabicyclo[5.3.0]decan-3-S-carbonsäure

oder dessen physiologisch unbedenklichem Salz.

6. Verfahren gemäss Anspruch 1 oder 2 zur Herstellung von

2-[Nα-(1-S-Carboxy-3-phenyl-propyl)-S-lysyl]-cis, endo-2-azabicyclo[5.3.0]decan-3-S-carbonsäure

oder dessen physiologisch unbedenklichem Salz.

7. Verfahren gemäss Anspruch 1 oder 2 zur Herstellung von

2-[N-(1-S-Carbethoxy-3-phenyl-propyl)-O-ethyl-S-tyrosyl]-cis,endo-2-azabicyclo[5.3.0]decan-3-S-carbonsäure

oder dessen physiologisch unbedenklichem Salz.

8. Verfahren gemäss Anspruch 1 oder 2 zur Herstellung von

2-[N-(1-S-Carbethoxy-3-phenyl-propyl)-O-methyl-S-tyrosyl]-cis,endo-2-azabicyclo[5.3.0]decan-3-S-carbonsäure

oder dessen physiologisch unbedenklichem Salz.

9. Verfahren zur Herstellung eines Mittels enthaltend eine Verbindung der Formel I gemäss einem der Ansprüche 1–8, dadurch gekennzeichnet, dass man diese in eine geeignete Zubereitungsform bringt.

10. Verfahren gemäss einem der Ansprüche 1–8 zur Herstellung einer Verbindung der Formel I zur Verwendung als Heilmittel.

11. Verfahren zur Herstellung von Verbindungen der Formeln IIIa und/oder IIIb in welchen W Wasserstoff, eine veresternde Gruppe, vorzugsweise Alkyl mit 1–6 C-Atomen oder Aralkyl mit 7 bis 8 C-Atomen bedeutet sowie deren Salze mit Säuren und (für den Fall W = Wasserstoff) mit Basen dadurch gekennzeichnet, dass Enamine der Formel VI, in welcher $X^1$ für Dialkylamino mit 2 bis 10 C-Atomen oder für einen Rest der Formel VII, worin m und o eine ganze Zahl von 1 bis 3, (m + o) ⩾ 3 und A $CH_2$, NH, O oder S bedeuten, steht

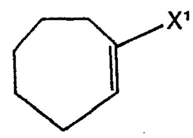

(VI)

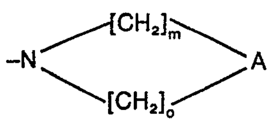

(VII)

mit N-acylierten β-Halogen-α-amino-carbonsäureestern der Formel VIII, in welcher $X^2$ für eine nucleofuge Gruppe, $Y^1$ für Alkanoyl mit 1 bis 5 C-Atomen, Aroyl mit 7 bis 9 C-Atomen oder andere, in der Peptidchemie übliche, sauer abspaltbare Schutzgruppen und $R^2$ für Alkyl mit 1 bis 5 C-Atomen ider Aralkyl mit 7 bis 9 C-Atomen steht

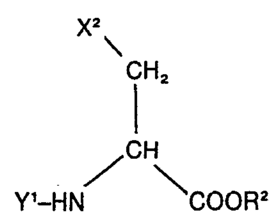

(VIII)

oder mit Acrylsäureestern der Formel IX, in welcher $Y^1$ und $R^2$ vorstehende Bedeutung haben

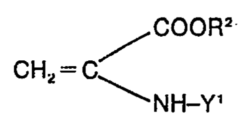

(IX)

zu Verbindungen der Formel X, in welcher $R^2$ und $Y^1$ vorstehende Bedeutung haben, umsetzt,

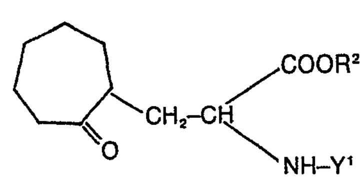

(X)

diese mit Hilfe starker Säuren unter Acylamid- und Esterspaltung zu Verbindungen der Formel XIa oder b cyclisiert,

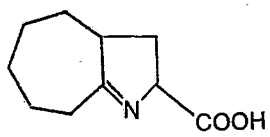

a

(XI)

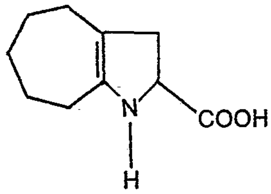

b

diese durch katalytische Hydrierung in Gegenwart von Übergangsmetallkatalysatoren oder durch Reduktion mit Boran-Aminkomplexen oder komplexen Borhydriden in niederen Alkoholen in Verbindungen der Formeln IIIa und/oder IIIb, in welchen W für Wasserstoff steht, überführt und gegebenenfalls zu Verbindungen der Formeln IIIa und/oder IIIb, in welchen W für eine veresternde Gruppe, vorzugsweise Alkyl mit 1 bis 6 C-Atomen oder Aralkyl mit 7 bis 8 C-Atomen steht, verestert und die so erhaltenen Verbindungen der Formel IIIa und/oder IIIb gegebenenfalls in ihre Salze überführt.

12. Verfahren gemäss Anspruch 11, dadurch gekennzeichnet, dass in den Formeln IIIa und b W Wasserstoff, tert.-Butyl, Benzyl oder Nitrobenzyl bedeutet.

**Claims for the Contracting States: DE, CH, DE, FR, GB, IT, LI, LU, NL, SE**

1. A compound of the formula I

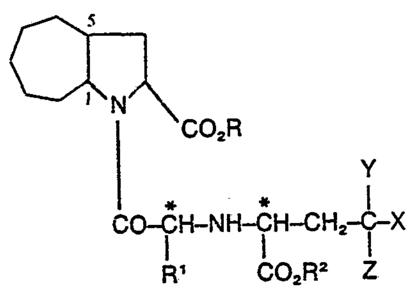

(I)

in which the hydrogen atoms on the bridgehead C atoms 1 and 5 have the cis-configuration relative to one another and the $CO_2R$ group on C atom 3 is oriented endo to the bicyclic ring system and in which

R denotes hydrogen ($C_1$–$C_6$)-alkyl, ($C_2$–$C_6$)-alkenyl or ($C_6$–$C_{12}$)-aryl-($C_1$–$C_4$)-alkyl,

$R^1$ denotes hydrogen, allyl, vinyl or a side chain of an optionally protected, naturally occurring α-aminoacid $R^1$–$CH(NH_2)$–COOH,

$R^2$ denotes hydrogen, ($C_1$–$C_6$)-alkyl, ($C_2$–$C_6$)-alkenyl or ($C_6$–$C_{12}$)-aryl-($C_1$–$C_4$)-alkyl,

Y denotes hydrogen or hydroxyl,

Z denotes hydrogen or

Y and Z together denote oxygen,

X denotes ($C_1$–$C_6$)-alkyl, ($C_2$–$C_6$)-alkenyl, ($C_5$–$C_9$)-cycloalkyl, ($C_6$–$C_{12}$)-aryl, preferably phenyl, which can be substituted once, twice or three times by ($C_1$–$C_4$)-alkyl, ($C_1$–$C_4$)-alkoxy, hydroxyl, halogen, nitro, amino, ($C_1$–$C_4$)-Alkylamino, di-($C_1$–$C_4$)-alkylamino and/or methylene-dioxy, or denotes indol-3-yl, and its physiologically acceptable salts.

2. A compound of the formula I as claimed in claim 1 in which

R denotes hydrogen,

$R^1$ denotes methyl, the optionally acylated side chain of lysine or the O-alkylated side chain of tyrosine,

$R^2$ denotes hydrogen, methyl, ethyl, benzyl or tert.-butyl,

X denotes phenyl or phenyl substituted once or twice with fluorine and/or chlorine,

Y denotes hydrogen or hydroxyl and

Z denotes hydrogen or

Y and Z together denote oxygen.

3. N-(1-S-Carboethoxy-3-phenylpropyl-S-alanyl-cis,endo-2-azabicyclo[5.3.0]decane-3-S-carboxylic acid

and its physiologically acceptable salts.

4. N-(1-S-Carboxy-3-phenylpropyl)-S-alanyl-cis,endo-2-azabicyclo[5.3.0]decane-3-S-carboxylic acid

and its physiologically acceptable salts.

5. N-(1-S-Carboethoxy-3-phenylpropyl)-S-lysyl-cis,endo-2-azabicyclo[5.3.0]decane-3-S-carboxylic acid

and its physiologically acceptable salts.

6. N-(1-S-Carboxy-3-phenylpropyl)-S-lysyl-cis, endo-2-azabicyclo[5.3.0]decane-3-S-carboxylic acid

and its physiologically acceptable salts.

7. N-(1-S-Carboethoxy-3-phenylpropyl)-O-ethyl-S-tyrosyl-cis,endo-2-azabicyclo[5.3.0]decane-3-S-carboxylic acid

and its physiologically acceptable salts.

8. N-(1-S-Carboethoxy-3-phenylpropyl)-O-methyl-S-tyrosyl-cis,endo-2-azabicyclo[5.3.0]decane-3-S-carboxylic acid

and its physiologically acceptable salts.

9. A process for the preparation of compounds as claimed in claim 1, which comprises reacting a compound of the formula II

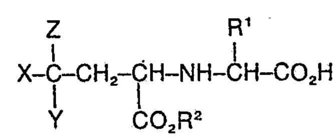

(II)

in which X, Y, Z, $R^1$ and $R^2$ have the meanings defined in claim 1, with the exception of $R^2$ = hydrogen, with a compound of the formulae IIIa or IIIb or the racemate

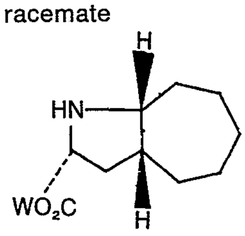

(IIIa)

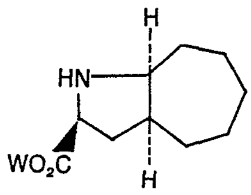

(IIIb)

in which W denotes hydrogen or a group esterifying carboxyl, if W denotes hydrogen, with preceding esterification of the compound of the formulae IIIa or IIIb or of the racemate, optionally converting the compounds of the formula I thus obtained by hydrogenation, treatment with acid and/or base into compounds of the formula I (R and/or $R^2$ = H), optionally esterifying the compounds thus obtained in a manner known per se and/or optionally converting them into their physiologically acceptable salts.

10. An agent comprising or containing a compound as claimed in any of claims 1–8.

11. A compound as claimed in any of claims 1 to 8 for use as a medicament.

12. A compound as claimed in any of claims 1 to 8 for use as a medicament for treating high blood pressure.

13. A compound as claimed in claim 11 or 12 for use in combination with a diuretic.

14. A compound of the formulae IIIa and/or IIIb in which W denotes hydrogen or an esterifying group preferably alkyl having 1–6 carbon atoms or aralkyl having 7 or 8 carbon atoms, and its salts with acids and (in the case where W = hydrogen) with bases.

15. A compound as claimed in claim 14, wherein, in formulae IIIa and b, W denotes hydrogen, tert.-butyl, benzyl or nitrobenzyl.

16. A process for the preparation of compounds of the formulae IIIa and/or IIIb as claimed in claim 14 or 15, which comprises reacting enamines of the formula VI in which $X^1$ represents dialkylamino having 2 to 10 carbon atoms or represents a radical of the formula VII, wherein m and o denote a whole number from 1 to 3, (m + o) ⩾ 3 and A denotes $CH_2$, NH, O or S

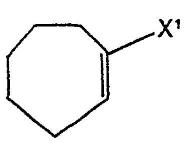

(VI)

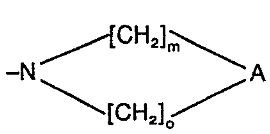

(VII)

with N-acylated β-halogeno-α-aminocarboxylic esters of the formula VIII, in which $X^2$ represents a nucleofugic group, $Y^1$ represents alkanoyl having 1 to 5 carbon atoms, aroyl having 7 to 9 carbon atoms or other protective groups which can be split off with acid and are customary in peptide chemistry, and $R^2$ represents alkyl having 1 to 5 carbon atoms or aralkyl having 7 to 9 carbon atoms,

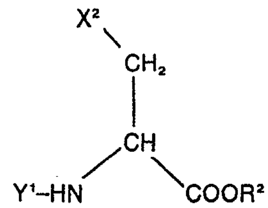

(VIII)

or with acrylic esters of the formula IX in which $Y^1$ and $R^2$ have the above meaning

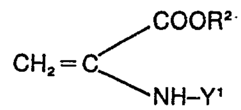

(IX)

to give compounds of the formula X in which $R^2$ and $Y^1$ have the above meaning,

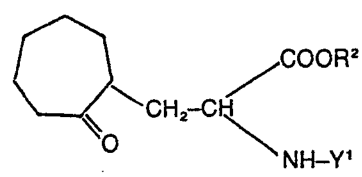

(X)

cyclizing the latter using strong acids, with cleavage of the acylamide and ester, to give compounds of the formula XIa or b,

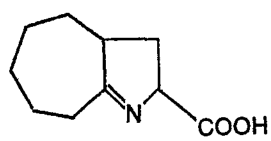

a

(XI)

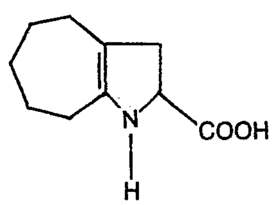

b

converting the latter by catalytic hydrogenation in the presence of transition metal catalysts or by reduction with borane-amine complexes or complex borohydrides in lower alcohols into compounds of the formulae IIIa and/or IIIb in which W represents hydrogen, optionally esterifying to give compounds of the formulaee IIIa and/or IIIb in which W represents alkyl having 1 to 6 carbon atoms or aralkyl having 7 to 8 carbon atoms, and optionally converting the compounds of the formula IIIa and/or IIIb thus obtained into their salts.

**Claims for the Contracting State: AT**

1. A process for the preparation of compounds of the formula I

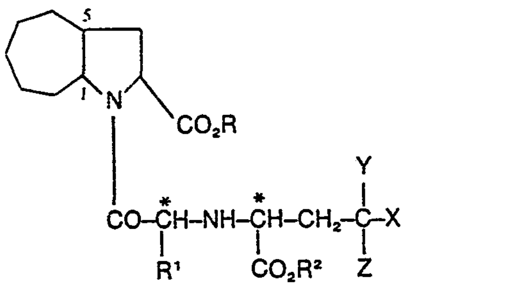

(I)

in which the hydrogen atoms on the bridgehead C atoms 1 and 5 have the cis-configuration relative to one another and the $CO_2R$ group on C atom 3 is oriented endo to the bicyclic ring system and in which

R denotes hydrogen ($C_1$–$C_6$)-alkyl, ($C_2$–$C_6$)-alkenyl or ($C_6$–$C_{12}$)-aryl-($C_1$–$C_4$)-alkyl,

$R^1$ denotes hydrogen, allyl, vinyl or a side chain of an optionally protected, naturally occurring α-aminoacid $R^1$–CH($NH_2$)–COOH,

$R^2$ denotes hydrogen, ($C_1$–$C_6$)-alkyl, ($C_2$–$C_6$)-alkenyl or ($C_6$–$C_{12}$)-aryl-($C_1$–$C_4$)-alkyl,

Y denotes hydrogen or hydroxyl,

Z denotes hydrogen or

Y and Z together denote oxygen,

X denotes ($C_1$–$C_6$)-alkyl, ($C_2$–$C_6$)-alkenyl, ($C_5$–$C_9$)-cycloalkyl, ($C_6$–$C_{12}$)-aryl, preferably phenyl, which can be substituted once, twice or three times by ($C_1$–$C_4$)-alkyl, ($C_1$–$C_4$)-alkoxy, hydroxyl, halogen, nitro, amino, ($C_1$–$C_4$)-alkylamino, di-($C_1$–$C_4$)-alkylamino and/or methylene-dioxy, or denotes indol-3-yl, and its physiologically acceptable salts, which comprises reacting a compound of the formula II

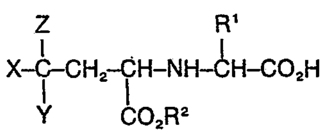

(II)

in which X, Y, Z, $R^1$ and $R^2$ have the meanings defined in claim 1, with the exception of $R^2$ = hydrogen, with a compound of the formulae IIIa or IIIb or the racemate

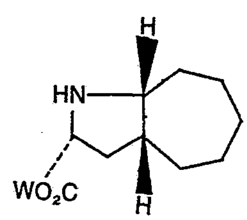

(IIIa)

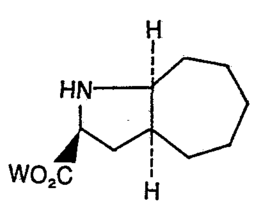

(IIIb)

in which W denotes hydrogen or a group esterifying carboxyl, if W denotes hydrogen, with preceding esterification of the compound of the formulae IIIa or IIIb or of the racemate, optionally converting the compounds of the formula I thus obtained by hydrogenation, treatment with acid and/or base into compounds of the formula I (R and/or $R^2$ = H), optionally esterifying the compounds thus obtained in a manner known per se and/or optionally converting them into their physiologically acceptable salts.

2. A process as claimed in claim 1 for preparing a compound of the formula I in which

R denotes hydrogen,

$R^1$ denotes methyl, the optionally acylated side chain of lysine or the O-alkylated side chain of tyrosine,

$R^2$ denotes hydrogen, methyl, ethyl, benzyl or tert.-butyl,

X denotes phenyl or phenyl substituted once or twice with fluorine and/or chlorine,

Y denotes hydrogen or hydroxyl and

Z denotes hydrogen or

Y and Z together denote oxygen, and its physiologically acceptable salts.

3. A process as claimed in claim 1 or 2 for preparing

N-(1-S-carboethoxy-3-phenylpropyl)-S-alanyl-cis, endo-2-azabicyclo[5.3.0]decane-3-S-carboxylic acid

or its physiologically acceptable salt.

4. A process as claimed in claim 1 or 2 for preparing

N-(1-S-carboxy-3-phenylpropyl)-S-alanyl-cis, endo-2-azabicyclo[5.3.0]decane-3-S-carboxylic acid

or its physiologically acceptable salt.

5. A process as claimed in claim 1 or 2 for preparing

N-(1-S-carboethoxy-3-phenylpropyl)-S-lysyl-cis, endo-2-azabicyclo[5.3.0]decane-3-S-carboxylic acid

or its physiologically acceptable salt.

6. A process as claimed in claim 1 or 2 preparing

N-(1-S-carboxy-3-phenylpropyl)-S-lysyl-cis,endo-2-azabicyclo[5.3.0]decane-3-S-carboxylic acid

or its physiologically acceptable salt.

7. A process as claimed in claim 1 or 2 preparing

N-(1-S-carboethoxy-3-phenylpropyl)-O-ethyl-S-tyrosyl-cis,endo-2-azabicyclo[5.3.0]decane-3-S-carboxylic acid

or its physiologically acceptable salt.

8. A process as claimed in claim 1 or 2 preparing

N-(1-S-carboethoxy-3-phenylpropyl)-O-methyl-S-tyrosyl-cis,endo-2-azabicyclo[5.3.0]decane-3-S-carboxylic acid

or its physiologically acceptable salt.

9. A process for preparation an agent containing a compound of the formula I as claimed in any of claims 1–8, characterized in that the compound is brought in a suitable form for administration.

10. A process according to any of claims 1–8 for preparing a compound of the formula I for use as a medicament.

11. A process for the preparation of compounds of the formulae IIIa and/or IIIb which comprises

reacting enamines of the formula VI in which $X^1$ represents dialkylamino having 2 to 10 carbon atoms or represents a radical of the formula VII, wherein m and o denote a whole number from 1 to 3, $(m + o) \geqslant 3$ and A denotes $CH_2$, NH, O or S

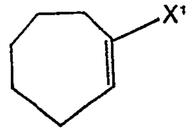

(VI)

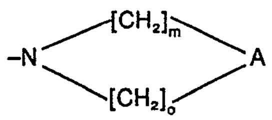

(VII)

with N-acylated β-halogeno-α-aminocarboxylic esters of the formula VIII, in which $X^2$ represents a nucleofugic group, $Y^1$ represents alkanoyl having 1 to 5 carbon atoms, aroyl having 7 to 9 carbon atoms or other protective groups which can be split off with acid and are customary in peptide chemistry, and $R^2$ represents alkyl having 1 to 5 carbon atoms or aralkyl having 7 to 9 carbon atoms,

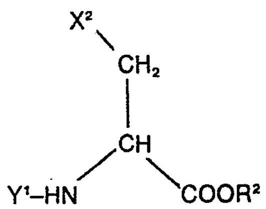

(VIII)

or with acrylic esters of the formula IX in which $Y^1$ and $R^2$ have the above meaning

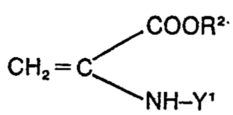

(IX)

to give compounds of the formula X in which $R^2$ and $Y^1$ have the above meaning,

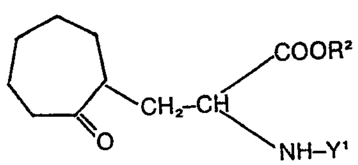

(X)

cyclizing the latter using strong acids, with cleavage of the acylamide and ester, to give compounds of the formula XIa or b,

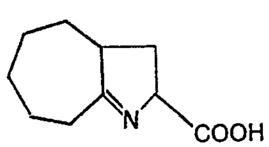

a

(XI)

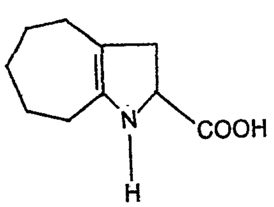

b

converting the latter by catalytic hydrogenation in the presence of transition metal catalysts or by reduction with borane-amine complexes or complex borohydrides in lower alcohols into compounds of the formulae IIIa and/or IIIb in which W represents hydrogen, optionally esterifying to give compounds of the formulae IIIa and/or IIIb in which W represents alkyl having 1 to 6 carbon atoms or aralkyl having 7 to 8 carbon atoms, and optionally converting the compounds of the formula IIIa and/or IIIb thus obtained into their salts.

12. A process as claimed in claim 11, characterized in that in formulae IIIa and b W denotes hydrogen, tert.-butyl, benzyl or nitrobenzyl.

**Revendications pour les Etats contractants: BE, CH, DE, FR, GB, IT, LI, LU, NL, SE**

1. Composé de formule I

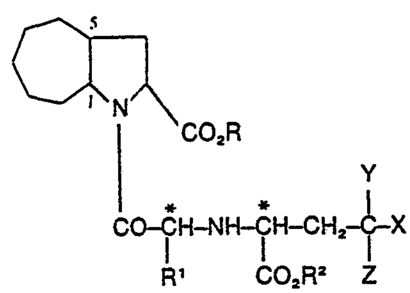

(I)

dans laquelle les atomes d'hydrogène sur les atomes de carbone têtes de pont 1 et 5 sont en configuration cis l'un par rapport à l'autre et le groupe $CO_2R$ sur l'atome de carbone 3 a une orientation endo par rapport au système bicyclique, et dans laquelle formule:

R représente l'hydrogène, un groupe alcoyle en $C_1$–$C_6$; alcényle en $C_2$–$C_6$ ou aryl($C_6$–$C_{12}$)-alcoyle en $C_1$–$C_4$;

$R^1$ représente l'hydrogène, un groupe allyle, vinyle ou une chaîne latérale d'un acide α-aminé naturel, $R^1$-$CH(NH_2)$-COOH, éventuellement protégé,

$R^2$ représente l'hydrogène, un groupe alcoyle en $C_1$–$C_6$, alcényle en $C_2$–$C_6$ ou aryl($C_6$–$C_{12}$)-alcoyle en $C_1$–$C_4$;

Y représente l'hydrogène ou un groupe hydroxy;

Z représente l'hydrogène ou

Y et Z représentent ensemble l'oxygène,

X représente un groupe alcoyle en $C_1$–$C_6$, alcényle en $C_2$–$C_6$, cycloalcoyle en $C_5$–$C_9$, aryle en $C_6$–$C_{12}$, qui peut être mono-, di- ou trisubstitué par un groupe alcoyle en $C_1$–$C_4$, alcoxy en $C_1$–$C_4$, hydroxy, halogéno, nitro, amino, alcoyl($C_1$–$C_4$)-amino, dialcoyl($C_1$–$C_4$)-amino et/ou méthylène-dioxy;

ou un groupe indol-3-yle;

ainsi que ses sels physiologiquement acceptables.

2. Composé de formule I dans lequel

R représente l'hydrogène,

$R^1$ représente un groupe méthyle, la chaîne

latérale éventuellement acylée de la lysine ou la chaîne latérale O-alcoylée (en $C_1$–$C_6$) de la tyrosine,

$R^2$ représente l'hydrogène, un groupe méthyle, éthyle, benzyle ou butyle tertiaire,

X représente un groupe phényle ou un groupe phényle mono- ou disubstitué par le fluor et/ou le chlore,

Y représente l'hydrogène ou un groupe hydroxy,

Z représente l'hydrogène ou

Y et Z représentent ensemble l'oxygène.

3. L'acide 2-[N-(1-S-carbéthoxy-3-phénylpropyl)-S-alanyl]-cis,endo-2-azabicyclo-[5.3.0]-décane-3-S-carboxylique,

ainsi que ses sels physiologiquement acceptables.

4. L'acide 2-[N-(1-S-carboxy-3-phényl-propyl)-S-alanyl]-cis,endo-2-azabicyclo-[5.3.0]-décane-3-S-carboxylique,

ainsi que ses sels physiologiquement acceptables.

5. L'acide 2-[Nα-(1-S-carbéthoxy-3-phénylpropyl)-S-lysyl]-cis,endo-2-azabicyclo-[5.3.0]-décane-3-S-carboxylique,

ainsi que ses sels physiologiquement acceptables.

6. L'acide 2-[Nα-(1-S-carboxy-3-phényl-propyl)-S-lysyl]-cis,endo-2-azabicyclo-[5.3.0]-décane-3-S-carboxylique,

ainsi que ses sels physiologiquement acceptables.

7. L'acide 2-[N-(1-S-carbéthoxy-3-phénylpropyl)-O-éthyl-S-tyrosyl]-cis,endo-2-azabicyclo-[5.3.0]-décane-3-S-carboxylique,

ainsi que ses sels physiologiquement acceptables.

8. L'acide 2-[N-(1-S-carbéthoxy-3-phénylpropyl)-O-méthyl-S-tyrosyl]-cis,endo-2-azabicyclo-[5.3.0]-décane-3-S-carboxylique,

ainsi que ses sels physiologiquement acceptables.

9. Un procédé pour la préparation de composés de formule I qui consiste à faire réagir un composé de formule II

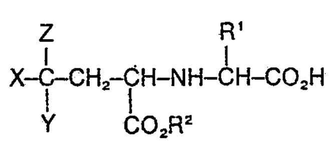

(II)

dans laquelle X, Y, Z, $R^1$ et $R^2$ ont les significations indiquées à la revendication 1, à l'exception de $R^2$ = hydrogène, avec un composé de formule IIIa ou IIIb ou le racémate

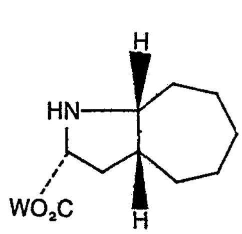

(IIIa)

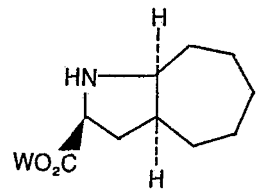

(IIIb)

dans lesquelles W représente l'hydrogène ou un groupe estérifiant le groupe carboxy, lorsque W représente l'hydrogène, après estérification du composé de formule IIIa ou IIIb ou du racémate, pour obtenir des composés de formule I, on convertit éventuellement ces composés par hydrogénation, traitement avec un acide et/ou une base en composés de formule I (R et/ou $R^2$ = H), on estérifie éventuellement d'une manière connue en soi les composés ainsi obtenus et/ou on les convertit éventuellement en leurs sels physiologiquement acceptables.

10. Composition constitué de, ou contenant un composé selon l'une quelconque des revendications 1 à 8.

11. Composé de formule I selon l'une quelconque des revendications 1 à 8 à utiliser comme medicament.

12. Composé de formule I selon l'une quelconque des revendications 1 à 8 à utiliser comme medicament pour le traitement de l'hypertension sanguine.

13. Composé de formule I selon la revendication 11 ou 12 à utiliser en association avec un diurétique.

14. Composé de formules IIIa et/ou IIIb, dans lequel W représente l'hydrogène ou un groupe estérifiant, de préférence un groupe alcoyle ayant de 1 à 6 atomes de carbone ou un groupe aralcoyle ayant 7 ou 8 atomes de carbone, ainsi que ses sels avec des acides et (dans le cas où W = hydrogène) avec des bases.

15. Composé tel que défini dans la revendication 14, caractérisé en ce que dans les formules IIIa et b W représente l'hydrogène, un groupe tert.-butyle, benzyle ou nitrobenzyle.

16. Un procédé pour la préparation de composés de formules IIIa et/ou IIIb, tels que définis aux points 14 et 15, caractérisé en ce qu'on fait réagir des énamines de formule VI, dans laquelle $X^1$ représente un groupe di-alcoylamino ayant de 2 à 10 atomes de carbone ou un radical de formule VII dans lequel m et o sont des nombres entiers compris entre 1 et 3, (m + o) ⩾ 3, et A représente $CH_2$, NH, O ou S

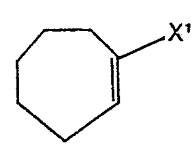

(VI)

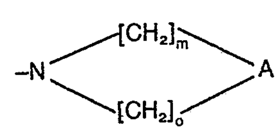

(VII)

avec des esters d'acides β-halogéno-α-aminocarboxyliques N,acylés, de formule VIII, dans les-

quels $X^2$ représente un groupe nucléofuge, $Y^1$ représente un groupe alcanoyle ayant de 1 à 5 atomes de carbone, aroyle ayant de 7 à 9 atomes de carbone ou d'autres groupes protecteurs éliminables par un acide, usuels dans la chimie des peptides, et $R^2$ représente un groupe alcoyle ayant de 1 à 5 atomes de carbone ou aralcoyle ayant de 7 à 9 atomes de carbone,

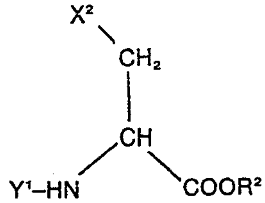

(VIII)

ou avec des esters de l'acide acrylique de formule IX, dans lesquels $Y^1$ et $R^2$ ont les significations précédentes

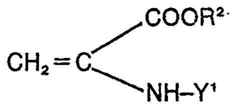

(IX)

pour obtenir des composés de formule X, dans lesquels $R^2$ et $Y^1$ ont les significations précédentes.

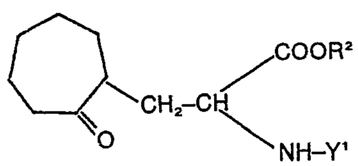

(X)

on cyclise ces composés à l'aide d'acides forts, avec scission des groupes acylamides et esters en des composés de formule XIa ou b,

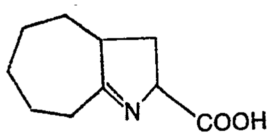

a

(XI)

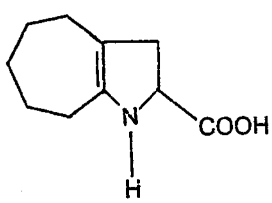

b

on convertit ces derniers par hydrogénation catalytique en présence de catalyseurs de métaux de transition ou par réduction avec des complexes de borane-amine ou des borohydrures complexes dans des alcools inférieurs, en des composés de formule IIIa ou IIIb, dans lesquels W représente l'hydrogène et éventuellement on estérifie ces derniers en des composés de formule IIIa ou IIIb dans lesquels W représente un groupe estérifiant, de préférence un groupe alcoyle ayant de 1 à 6 atoms de carbone ou aralcoyle ayant 7 ou 8 atomes de carbone, et on convertit éventuellement les composés ainsi obtenus de formule IIIa et/ou IIIb en leurs sels.

**Revendications pour l'Etat contractant AT**

1. Procédé de préparation de composés de formule I

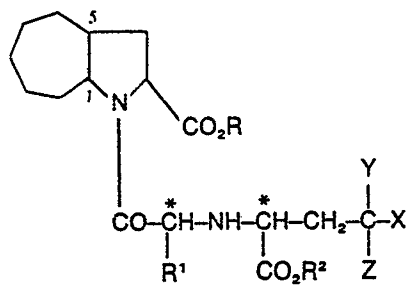

(I)

dans laquelle les atomes d'hydrogène sur les atomes de carbone têtes de pont 1 et 5 sont en configuration cis l'un par rapport à l'autre et le groupe $CO_2R$ sur l'atome de carbone 3 a une orientation endo par rapport au système bicyclique, et dans laquelle formule:

R représente l'hydrogène, un groupe alcoyle en $C_1$–$C_6$; alcényle en $C_2$–$C_6$ ou aryl($C_6$–$C_{12}$)-alcoyle en $C_1$–$C_4$;

$R^1$ représente l'hydrogène, un groupe allyle, vinyle ou une chaîne latérale d'un acide α-aminé naturel, $R^1$–CH($NH_2$)–COOH, éventuellement protégé,

$R^2$ représente l'hydrogène, un groupe alcoyle en $C_1$–$C_6$, alcényle en $C_2$–$C_6$ ou aryl($C_{6-12}$)-alcoyle en $C_1$–$C_4$;

Y représente l'hydrogène ou un groupe hydroxy;

Z représente l'hydrogène ou

Y et Z représentent ensemble l'oxygène,

X représente un groupe alcoyle en $C_1$–$C_6$, alcényle en $C_2$–$C_6$, cycloalcoyle en $C_5$–$C_9$, aryle en $C_6$–$C_{12}$, qui peut être mono-, di- ou trisubstitué par un groupe alcoyle en $C_1$–$C_4$, alcoxy en $C_1$–$C_4$, hydroxy, halogéno, nitro, amino, alcoyl($C_1$–$C_4$)-amino, dialcoyl($C_1$–$C_4$)-amino et/ou méthylènedioxy;

ou un groupe indol-3-yle;

ainsi que ses sels physiologiquement acceptables, caractérisé en ce qu'on fait réagir un composé de formule II

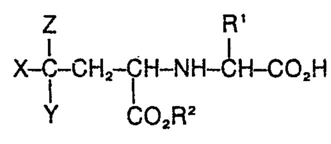

(II)

dans laquelle X, Y, Z, $R^1$ et $R^2$ ont les significations indiquées ci-dessus à l'exception de $R^2$ = hydrogène, avec un composé de formule IIIa ou IIIb ou le racémate

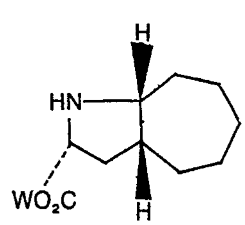

(IIIa)

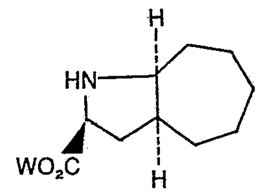
(IIIb)

dans lesquelles W représente l'hydrogène ou un groupe estérifiant le groupe carboxy, lorsque W représente l'hydrogène, après estérification du composé de formule IIIa ou IIIb ou du racémate, pour obtenir des composés de formule I, on convertit éventuellement ces composés par hydrogénation, traitement avec un acide et/ou une base en composés de formule I (R et/ou $R^2$ = H), on estérifie éventuellement d'une manière connue en soi les composés ainsi obtenus et/ou on les convertit éventuellement en leurs sels physiologiquement acceptables.

2. Procédé selon la revendication 1 de préparation d'un composé de formule I dans lequel

R représente l'hydrogène,

$R^1$ représente un groupe méthyle, la chaîne latérale éventuellement acylée de la lysine ou la chaîne latérale O-alcoylée (en $C_1$–$C_6$) de la tyrosine,

$R^2$ représente l'hydrogène, un groupe méthyle, éthyle, benzyle ou butyle tertiaire,

X représente un groupe phényle ou un groupe phényle mono- ou disubstitué par le fluor et/ou le chlore,

Y représente l'hydrogène ou un groupe hydroxy,

Z représente l'hydrogène ou

Y et Z représentent ensemble l'oxygène, ainsi que ses sels physiologiquement acceptables.

3. Procédé selon la revendication 1 ou 2 de préparation de l'acide
2-[N-(1-S-carbéthoxy-3-phényl-propyl)-S-alanyl]-cis,endo-2-azabicyclo-[5.3.0]-décane-3-S-carboxylique,
ainsi que ses sels physiologiquement acceptables.

4. Procédé selon la revendication 1 ou 2 de préparation de l'acide
2-[N-(1-S-carboxy-3-phényl-propyl)-S-alanyl]-cis,endo-2-azabicyclo-[5.3.0]-décane-3-S-carboxylique,
ainsi que ses sels physiologiquement acceptables.

5. Procédé selon la revendication 1 ou 2 de préparation de l'acide
2-[Nα-(1-S-carbéthoxy-3-phényl-propyl)-S-lysyl]-cis,endo-2-azabicyclo-[5.3.0]-décane-3-S-carboxylique,
ainsi que ses sels physiologiquement acceptables.

6. Procédé selon la revendication 1 ou 2 de préparation de l'acide
2-[Nα-(1-S-carboxy-3-phényl-propyl)-S-lysyl]-cis,endo-2-azabicyclo-[5.3.0]-décane-3-S-carboxylique,
ainsi que ses sels physiologiquement acceptables.

7. Procédé selon la revendication 1 ou 2 de préparation de l'acide
2-[N-(1-S-carbéthoxy-3-phényl-propyl)-O-éthyl-S-tyrosyl]-cis,endo-2-azabicyclo-[5.3.0]-décane-3-S-carboxylique,
ainsi que ses sels physiologiquement acceptables.

8. Procédé selon la revendication 1 ou 2 de préparation de l'acide
2-[N-(1-S-carbéthoxy-3-phényl-propyl)-O-méthyl-S-tyrosyl]-cis,endo-2-azabicyclo-[5.3.0]-décane-3-S-carboxylique,
ainsi que ses sels physiologiquement acceptables.

9. Procédé pour la préparation d'une composition contenant un composé de formule I selon l'une quelconque des revendications 1 à 8, consistant à mettre celle-ci sous une forme d'administration appropriée.

10. Procédé selon l'une quelconque des revendications 1 à 8 pour la préparation d'un composé de formule I à utiliser comme médicament.

11. Procédé pour la préparation de composés de formule IIIa et/ou IIIb, dans lesquelles W représente l'hydrogène, un groupe estérifiant, de préférence un groupe alcoyle ayant de 1 à 6 atomes de carbone ou un groupe aralcoyle ayant 7 ou 8 atomes de carbone, ainsi que leurs sels avec des acides et (dans le cas où W = hydrogène) avec des bases, caractérisé en ce qu'on fait réagir des énamines de formule VI dans lesquelles $X^1$ représente un groupe di-alcoylamino ayant de 2 à 10 atomes de carbone ou un radical de formule VII dans lequel m et o sont des nombres entiers compris entre 1 et 3, $(m + o) \geqslant 3$, et A représente $CH_2$, NH, O ou S.

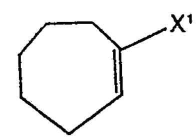
(VI)

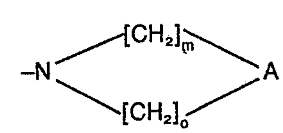
(VII)

avec des esters d'acides β-halogéno-α-aminocarboxyliques N,acylés, de formule VIII, dans lesquels $X^2$ représente un groupe nucléofuge, $Y^1$ représente un groupe alcanoyle ayant de 1 à 5 atomes de carbone, aroyle ayant de 7 à 9 atomes de carbone ou d'autres groupes protecteurs éliminables par un acide, usuels dans la chimie des peptides, et $R^2$ représente un groupe alcoyle ayant de 1 à 5 atomes de carbone ou aralcoyle ayant de 7 à 9 atomes de carbone,

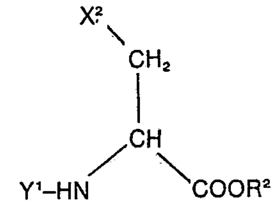
(VIII)

ou avec des esters de l'acide acrylique de formule IX, dans lesquels $Y^1$ et $R^2$ ont les significations précédentes

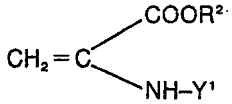

(IX)

pour obtenir des composés de formule X, dans lesquels $R^2$ et $Y^1$ ont les significations précédentes.

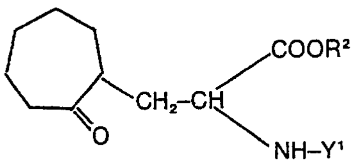

(X)

on cyclise ces composés à l'aide d'acides forts, avec scission des groupes acylamides et esters en des composés de formule XIa ou b,

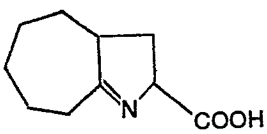

(XI)

a

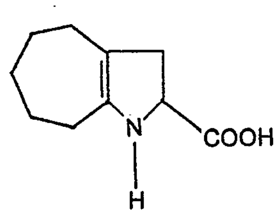

b

on convertit ces derniers par hydrogénation catalytique en présence de catalyseurs de métaux de transition ou par réduction avec des complexes de borane-amine ou des borohydrures complexes dans des alcools inférieurs, en des composés de formule IIIa et IIIb, dans lesquels W représente l'hydrogène et éventuellement on estérifie ces derniers en des composés de formule IIIa et IIIb dans lesquels W représente un groupe estérifiant, de préférence un groupe alcoyle ayant de 1 à 6 atomes de carbone ou aralcoyle ayant 7 ou 8 atomes de carbone, et on convertit éventuellement les composés ainsi obtenus de formule IIIa et/ou IIIb en leurs sels.

12. Procédé selon la revendication 11 caractérisé en ce que dans les formules IIIa et b W représente hydrogène, un groupe tert.-butyle, benzyle ou nitrobenzyle.